# EUROPEAN PATENT SPECIFICATION

(11) **EP 4 461 268 B1**
(45) Date of publication and mention of the grant of the patent: **28.10.2026**
(21) Application number: 24194790.2
(22) Date of filing: 23.11.2021
(51) Int. Cl.: A61B 17/15, A61B 17/17, A61F 2/46

(54) **PIVOTING TIBIAL RESECTION GUIDE AND ASSEMBLY**
SCHWENKBARE TIBIARESEKTIONSFÜHRUNG UND ANORDNUNG
GUIDE PIVOTANT DE RÉSECTION TIBIALE ET ENSEMBLE

(30) Priority: 07.12.2020 US 202063122115 P; 24.09.2021 US 202163248059 P; 22.11.2021 US 202117455945
(43) Date of publication of application: 13.11.2024
(62) Document number(s) of the earlier application(s) in accordance with Art. 76 EPC: 21841046.2 / 4 255 323
(73) Proprietor: MicroPort Orthopedics Holdings Inc., Arlington, TN 38002 (US)
(72) Inventor: HARRIS, Brian R. Jr., Cordova, TN 38016 (US); SIMES, Robert F. Jr., Hendersonville, NC 28739 (US)
(74) Representative: Novagraaf International SA

(56) References cited:
- US-A1- 2005 049 603

## Description

### BACKGROUND OF THE INVENTION

### 1. Technical Field

The present disclosure relates generally to the field of knee arthroplasties and more particularly to kinematic alignment tibial guide transfer instruments configured to accurately place components of an endoprosthetic knee implant.

### 2. Related Art

Knee arthroplasties are procedures in which an orthopedic surgeon replaces portions of severely diseased knee joints with an artificial endoprosthetic implant that is intended to restore joint function and alleviate pain. The procedure itself generally consists of the surgeon making a vertical midline anterior incision on the bent knee *(i.e.,* a knee in flexion). The surgeon then continues to incise tissue to access the joint capsule. After the joint capsule is pierced, the patella is moved out of the way and the distal condyles of the femur, the cartilaginous meniscus, and the proximal tibial plateau are exposed.

The surgeon then removes the cartilaginous meniscus and uses instrumentation to measure and resect the distal femur and proximal tibia independently from one another to accommodate the endoprosthetic knee implant. The resections themselves often remove areas of diseased bone and modify the bones' shapes to better accommodate complementary shapes of the respective implant components. That is, the resected distal femur will eventually fit into a complementary femoral implant component and the resected proximal tibia will eventually support a complementary tibial implant component. The surgeon selects from differently sized implant components to match the size of the patient's bones.

There are several schools of thought concerning the angles at which resection of the distal femoral condyles and the proximal tibia should be made. The angles of resection largely determine how the implant components will sit in the joint and can influence how the artificial joint will perform over time.

One such school of thought is the kinematic alignment philosophy. With kinematic alignment, the surgeon seeks to restore the natural pre-diseased joint line of the patient based on data made available to the surgeon both pre-operatively and intra-operatively.

It should come as no surprise that surgical approaches differ even among surgeons who practice kinematic alignment techniques. Some surgeons prefer to use calipers or other measurement instrumentation to measure dimensions of the distal femur and the proximal tibia independently from one another. This approach generally provides the greatest amount of autonomy, which in turn permits the greatest amount of subjectivity and variability in the placement of resection planes (and ultimately, the placement of the implant components). As such, this independently referencing approach can result in the greatest amount of trial and error.

This technique therefore generally prolongs the amount of time that a patient is under a general anesthetic. This technique also increases the risk that the final placement of the joint line will not align with the natural pre-diseased joint line precisely. In extreme cases, non-alignment may encourage supplemental or revision procedures that would have been avoidable otherwise. Even in cases that ultimately place the joint line perfectly, the amount of time required to calculate, resect, install, and test the kinematically aligned joint prolongs the time that the surgical area is exposed. While surgeons typically make every effort to maintain a sterile surgical environment, prolonged procedures nevertheless increase infection risk, prolong blood loss, and can result in more trauma to the surrounding tissue.

Other surgeons may use tools such as the ones disclosed in U.S. Pat. Pub. No. US 2019/0231365 to improve accuracy and to reduce operative times. While certainly an improvement, these tools preserve an element of subjectivity and the risks associated with subjectivity. Setting the tools up and properly adjusting them also adds additional steps to the procedure. U.S. Pub. No. 2005/0049603 to Calton, *et. al.* discloses a femoral knee block. In the aggregate, these additional steps may affect the number of patients that the surgeon can see in a day. Instruments with several moving parts can also increase the time needed to sterilize the instruments between procedures.

### SUMMARY OF THE INVENTION

As such, there is a long felt, but unresolved need to overcome the disadvantages of the prior art. It is contemplated that the instruments, assemblies, kits, systems, and methods disclosed herein can be used to overcome the disadvantages of the prior art.

The problems of imprecise placement of the resection planes in a knee replacement surgery and of increased procedure time associated with procedures that rely heavily on subjective placement of the resection planes are mitigated by a distally referencing linking drill guide assembly comprising: a linking drill guide comprising: a femoral portion, the femoral portion configured to engage a first femoral engagement member, a tibial portion, the tibial portion configured to engage a first tibial engagement member, and a body connecting the femoral portion to the tibial portion; and a femoral referencing instrument, the femoral referencing instrument having a first complimentary femoral engagement member, the first complimentary femoral engagement member being configured to engage the first femoral engagement member, wherein the distally referencing linking drill guide assembly has an engaged configuration and a disengaged configuration, wherein the engaged configuration comprises the first femoral engagement member engaging the first complimentary engagement member, and wherein the disengaged configuration comprises the first femoral engagement member not engaging the first complementary femoral engagement member. The claimed invention is defined in the appended claims.

It is contemplated that exemplary embodiments described herein can provide improved kinematic knee instruments and methods.

It is further contemplated that exemplary embodiments described herein can provide distal referencing options for transferring alignment to a tibial resection guide.

The foregoing objectives can be achieved by providing kinematic alignment tibial guide transfer instruments and methods having the features described herein.

The foregoing and other objects, features, aspects, and advantages of the invention will become more apparent from the following detailed description of the invention when considered in conjunction with the accompanying drawings.

### BRIEF DESCRIPTION OF THE DRAWINGS

The foregoing will be apparent from the following more particular description of exemplary embodiments of the disclosure, as illustrated in the accompanying drawings. The drawings are not necessarily to scale, with emphasis instead being placed upon illustrating the disclosed embodiments.
**FIGS. 1 - 10** generally depict method steps and exemplary devices and assemblies that include an exemplary linking drill guide and that can that involve the use of a femoral trial having complementary femoral engagement members.
**FIG. 1** depicts a femoral trial disposed on the distal end of a resected femur.
**FIG 2** depicts threaded femoral linking pins inserted through reference holes in the femoral trial shown in **FIG. 1****.**
**FIGS. 3A** and **3B** are perspective views of the elements of **FIG. 2** and further includes spoon gap spacers inserted medially and laterally between the femoral trial and the tibial plateau of a proximal tibia.
**FIG. 4** is a perspective view showing the elements from **FIGS. 3A** and **3B** and further depicts a linking drill guide slid over the femoral linking pins with the gap spacers present.
**FIG. 5A** and **5B** are perspective views of the elements of **FIG. 4** further showing tibial linking pins placed in the tibial reference holes in the linking drill guide.
**FIGS. 6A** and **6B** are perspective views depicting the elements of **FIGS. 5A** and 5B, except that snap-on spacers are disposed between the femoral trial and the tibial plateau instead of the spoon gap spacers.
**FIG. 7A** is a perspective view depicting the elements of **FIGS. 6A** and **6B****,** wherein the linking drill guide has been removed and a pivoting tibial resection guide has been slid onto the tibial linking pins.
**FIG. 7B** is a side view of the elements depicted in **FIG. 7A****.**
**FIG. 8** is another side view of the elements depicted in **FIG. 7A** in which the tibial posterior slope of the tibial resection guide has been adjusted to match the natural slope of the patient's tibia.
**FIG. 9** is a perspective view of the elements depicted in **FIG. 7A****,** wherein the tibial linking pins have been removed and the pivoting tibial resection guide is secured to the proximal tibial through securing pins in securing pin holes.
**FIG. 10** is a side view of the elements depicted in **FIG. 9****,** except that the femoral trial has been removed and proximal tibial resection has been performed through a slot in the pivoting tibial resection guide.
**FIGS. 11** - **15** generally depict method steps and exemplary devices and assemblies that include another embodiment of an exemplary linking drill guide and that can involve the use of a distal referencing guide having complementary femoral engagement members.

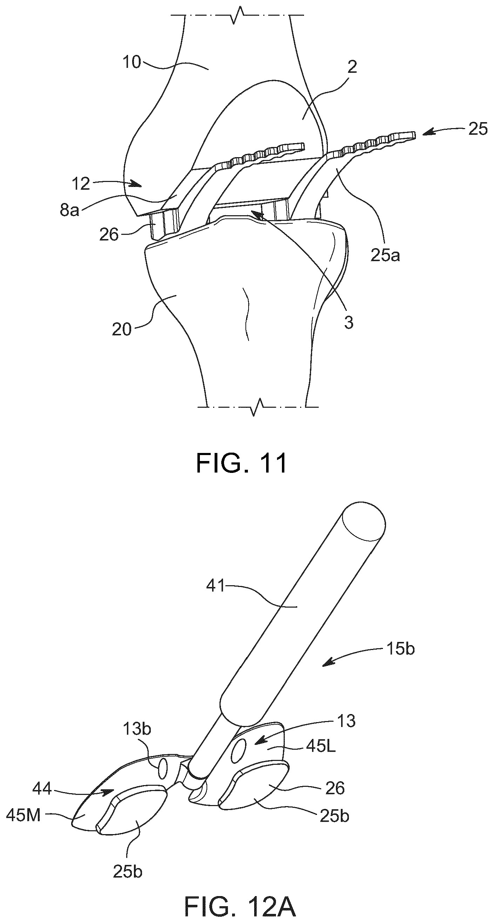

**FIG. 11** is a perspective view of a knee placed in extension, wherein the distal femur has been resected and distal referencing gap spacers have been inserted medially and laterally to fill a joint space between the resected femur and the intact tibia to determine the medial and lateral gap distance.
**FIG. 12A** is a perspective view of a distal referencing guide having a removable handle connected to a femoral portion comprising a medial condylar portion and a lateral condylar portion, wherein a distal portion of each of the condylar portions is configured to receive a snap on spacer.

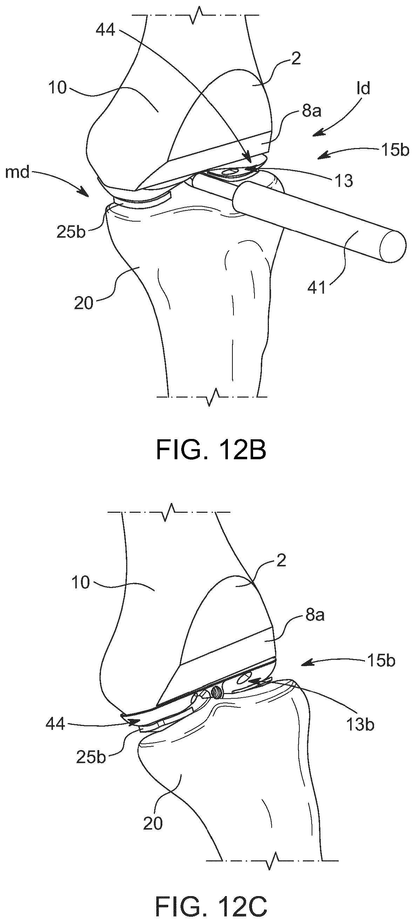

**FIG. 12B** is a perspective view depicting the distal referencing guide inserted into the gap.
**FIG. 12C** is a perspective view of the distal referencing guide disposed within the gap, wherein the handle has been removed.

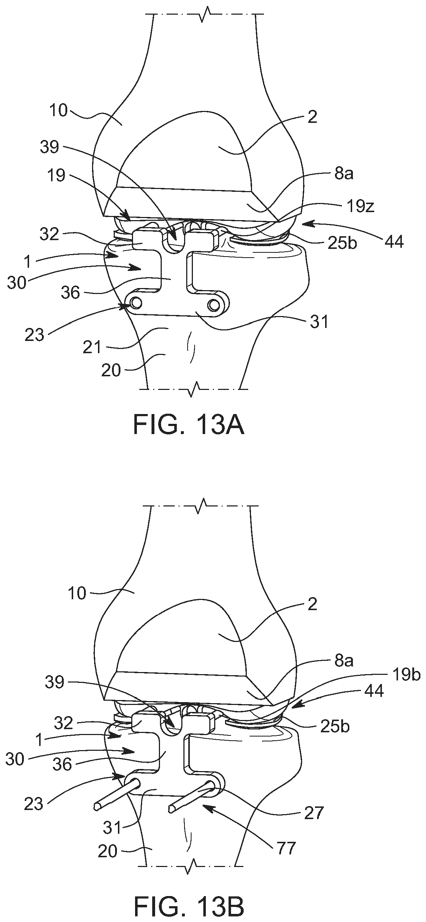

**FIG. 13A** is a perspective view showing the elements of **FIG. 12C** and further comprises an exemplary linking drill guide having orientation pins inserted into the condylar portions of the distal referencing guide.
**FIG. 13B** is a perspective view of the elements shown in **FIG. 13A** and further depicts tibial linking pins having been inserted into the tibial reference holes in the linking drill guide.

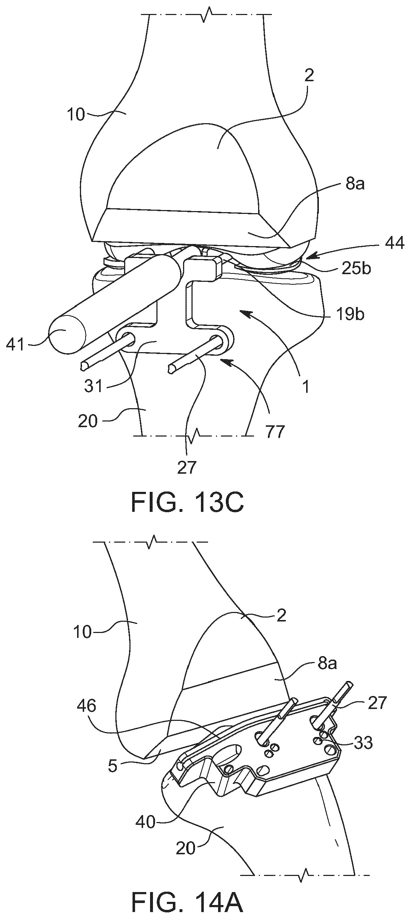

**FIG. 13C** is perspective view of the elements shown in **FIG. 13B** and further depicts the removable handle having been reattached to the femoral portion of the distal referencing guide.
**FIG. 14A** is a perspective view showing a pivoting tibial resection disposed over the tibial linking pins remaining in tibia, wherein the distal gap spacer assembly and the linking drill guide have been removed.

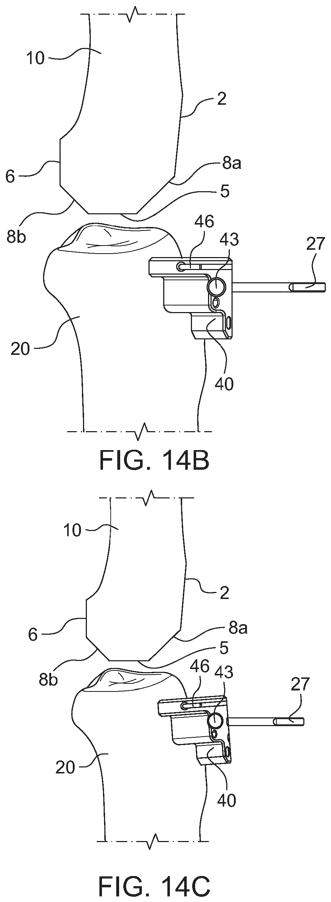

**FIG. 14B** is a side view of the elements shown in **FIG. 14A****.**
**FIG. 14C** is a side view of the elements depicted in **FIG. 14B** in which the tibial posterior slope of the tibial resection guide has been adjusted to match the natural slope of the patient's tibia.

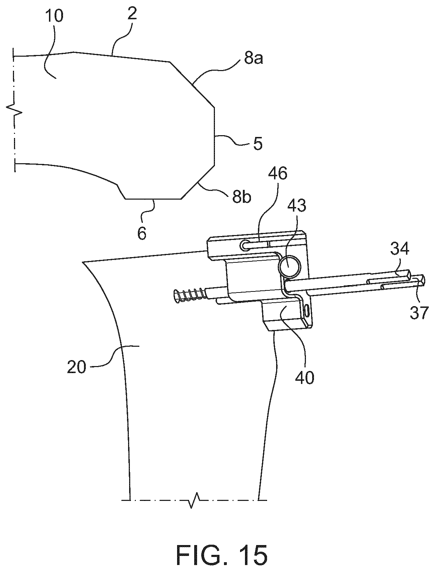

**FIG. 15** is a side view of the elements depicted in **FIG. 14C****,** except that the tibial linking pins have been removed, standard pins have been inserting into standard securing pin holes to fix the orientation of the pivoting tibial resection guide, and proximal tibial resection has been performed through a slot in the pivoting tibial resection guide.

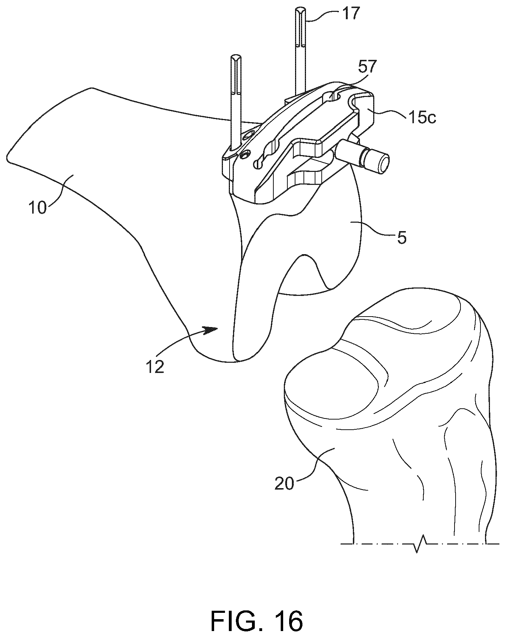

**FIGS. 16 - 28** generally depict method steps and exemplary devices and assemblies that include other embodiments of exemplary linking drill guide and that can that involve the use of a distal femoral resection guide.
**FIG. 16** is a perspective view of a knee is flexion wherein the distal femoral resection guide is oriented on the distal femur after the distal cut as been made.

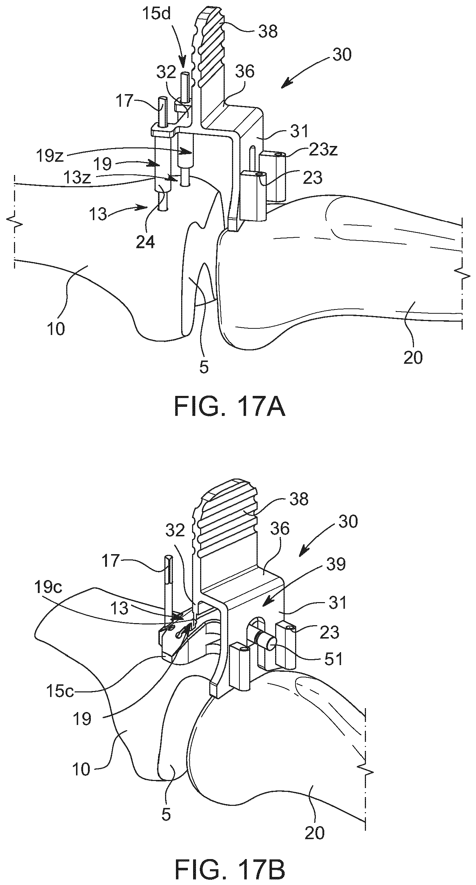

**FIG. 17A** is a perspective view of the knee is extension, wherein an exemplary embodiment of a linking drill guide is provided, wherein the exemplary linking drill guide is configured to be engaged to the pins previously used to secure the distal femoral resection guide in a resection orientation.
**FIG. 17B** is a perspective view of a knee in extension, wherein another exemplary embodiment of a linking drill guide comprising a blade configured to fit securely into the femoral resection slot of the distal femoral resection guide is provided.

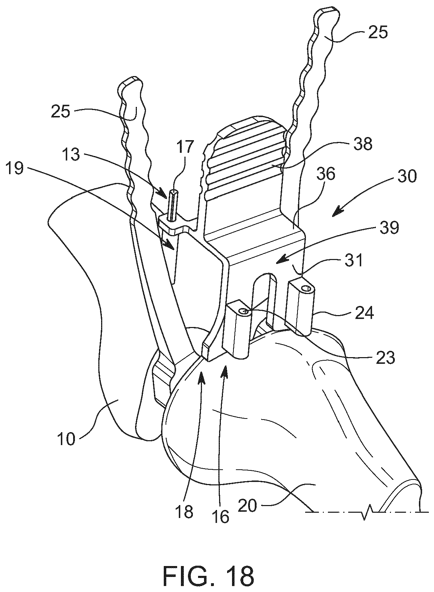

**FIG. 18** is a perspective view of a knee in extension in which gap spacers are inserted into the joint space between the resected distal femur and the proximal tibial, wherein an embodiment of the linking drill guide is provided.

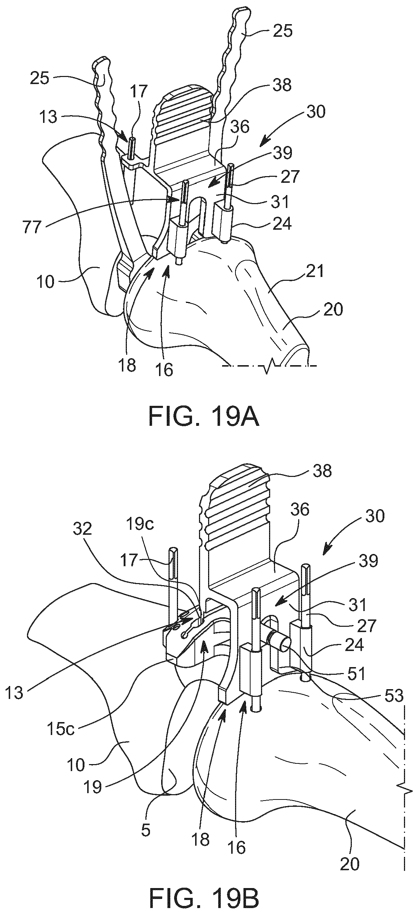

**FIG. 19A** is a perspective view of the elements depicted in **FIG. 18** and further comprises tibial linking pins extending into the tibia through the tibial portion of the linking drill guide.
**FIG. 19B** is a perspective view of the elements depicted in **FIG. 17B** and further comprises tibial linking pins extending into the tibia through the tibial portion of the linking drill guide.

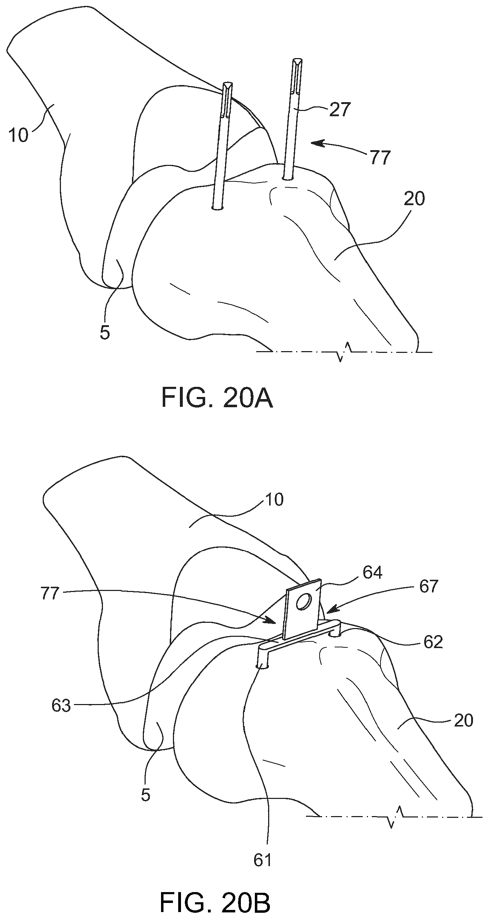

**FIG. 20A** is a perspective view of the tibial linking pins remaining in the tibia after all other elements have been removed.
**FIG. 20B** is a perspective view of an exemplary spike plate remaining in the bores left by the tibial linking pins after the tibial linking pins and all other elements have been removed.

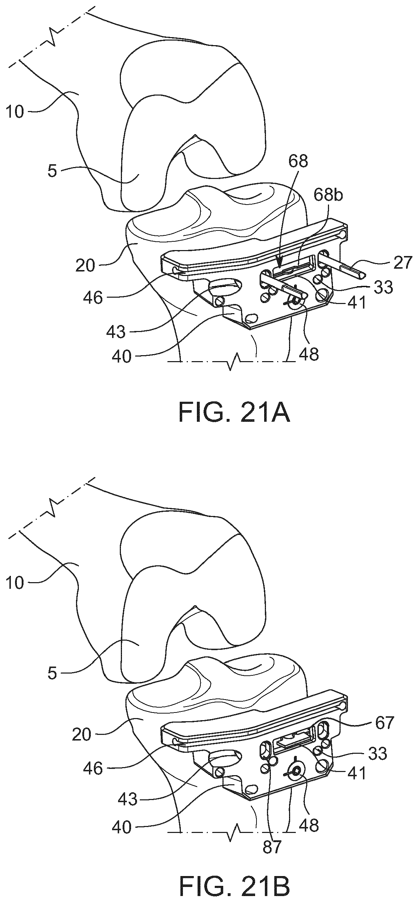

**FIG. 21A** is a perspective view of a pivoting tibial resection guide disposed upon the tibial linking pins depicted in **FIG. 20A****.**
**FIG. 21B** is a perspective view of a pivoting tibial resection guide disposed upon a linking tab of the spike plate depicted in **FIG. 20B.**

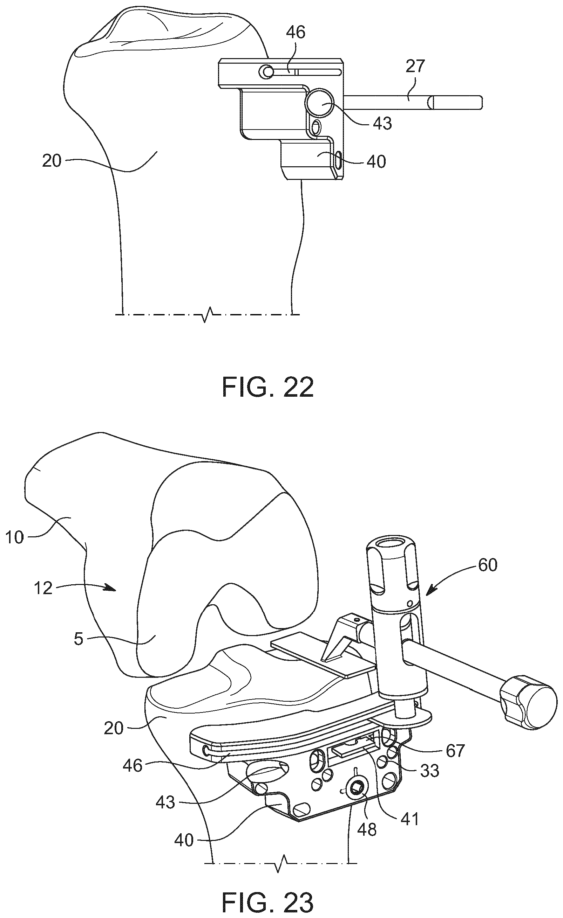

**FIG. 22** is a side view of the pivoting tibial resection guide disposed on tibial linking pins.
**FIG. 23** is a perspective view of the elements depicted in **FIG. 21B** and further comprising a tibial visual slope gauge with the locking cam in the unlocked position.

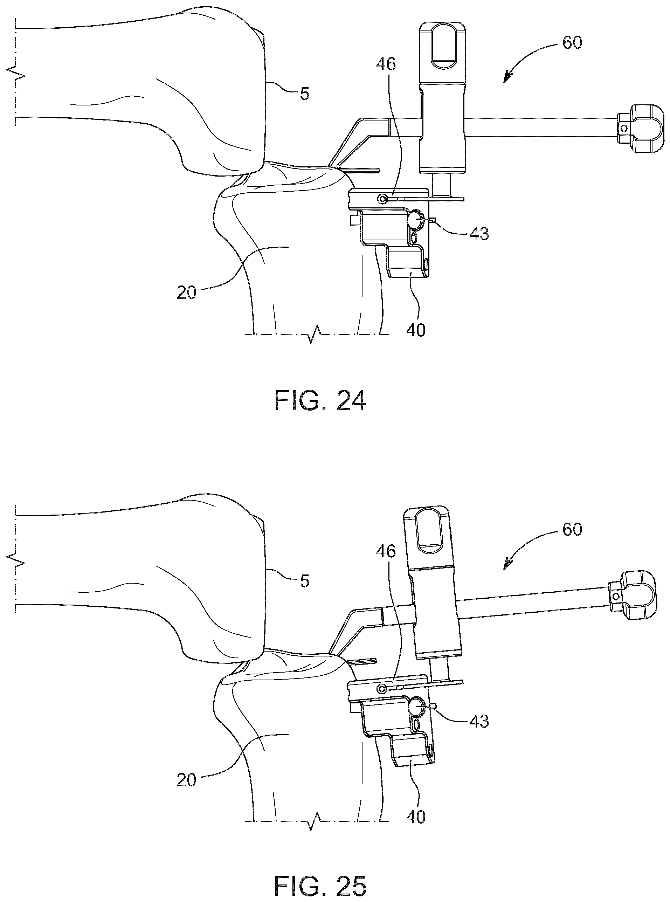

**FIG. 24** is a side view of the elements depicted in **FIG. 23****.**
**FIG. 25** is a side view of the elements depicted in **FIG. 23****,** wherein the pivoting resection guide **40** has been pivoted to adjust the posterior slope of the resection plane.

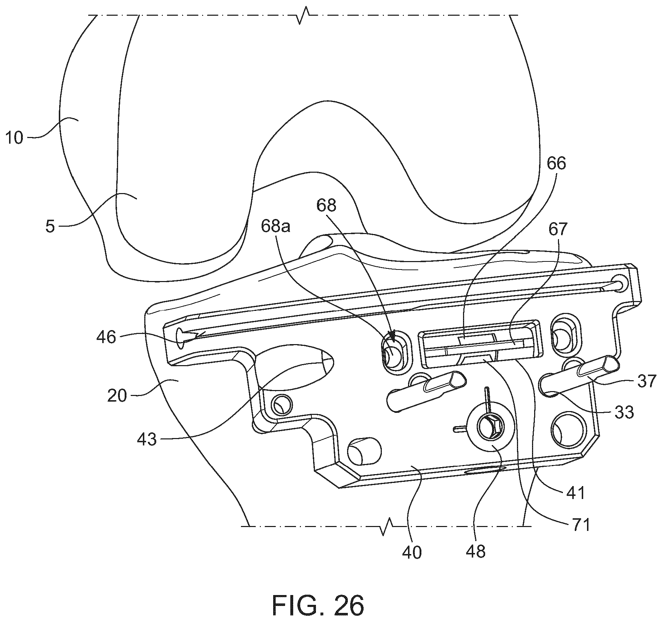

**FIG. 26** is a perspective view of the pivoting tibial resection guide disposed at the desired posterior slope with the locking cam in the locked position.

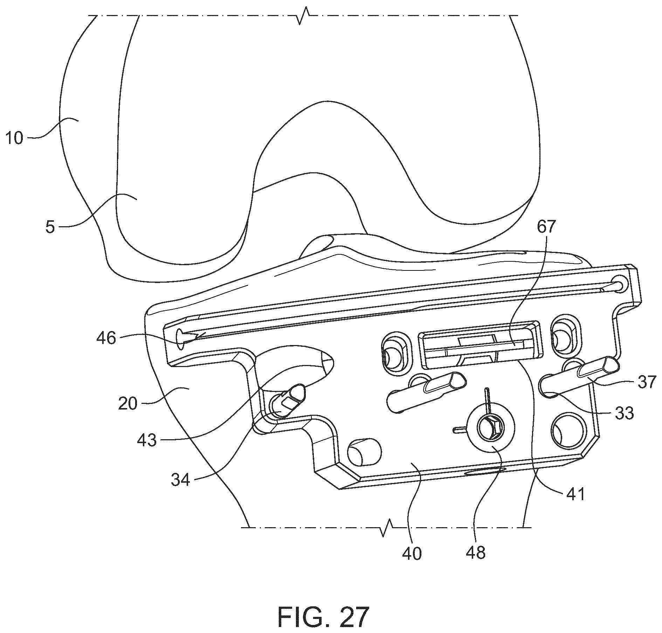

**FIG. 27** is a perspective view of the pivoting tibial resection guide disposed at the desired posterior slope and further depicts a divergent fixation pin extending through the pivoting tibial resection guide to further secure the pivoting tibial resection guide to the tibia at the desired slope.

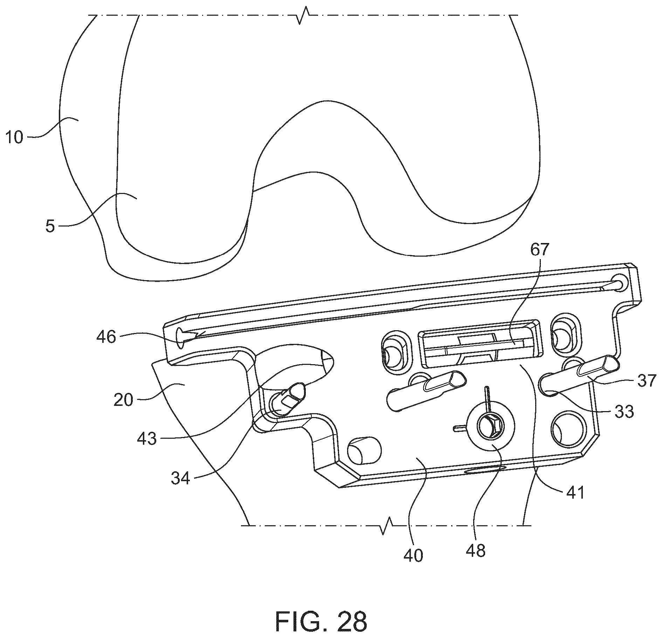

**FIG. 28** is a perspective view of the pivoting tibial resection guide disposed at the desired posterior slope, wherein the tibial plateau has been resected.

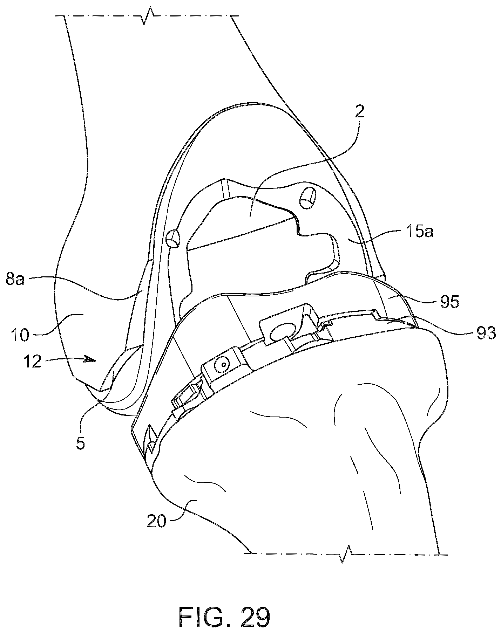

**FIG. 29** is a perspective view of a femoral trial, tibial base trial and meniscal insert based on sizing criteria.

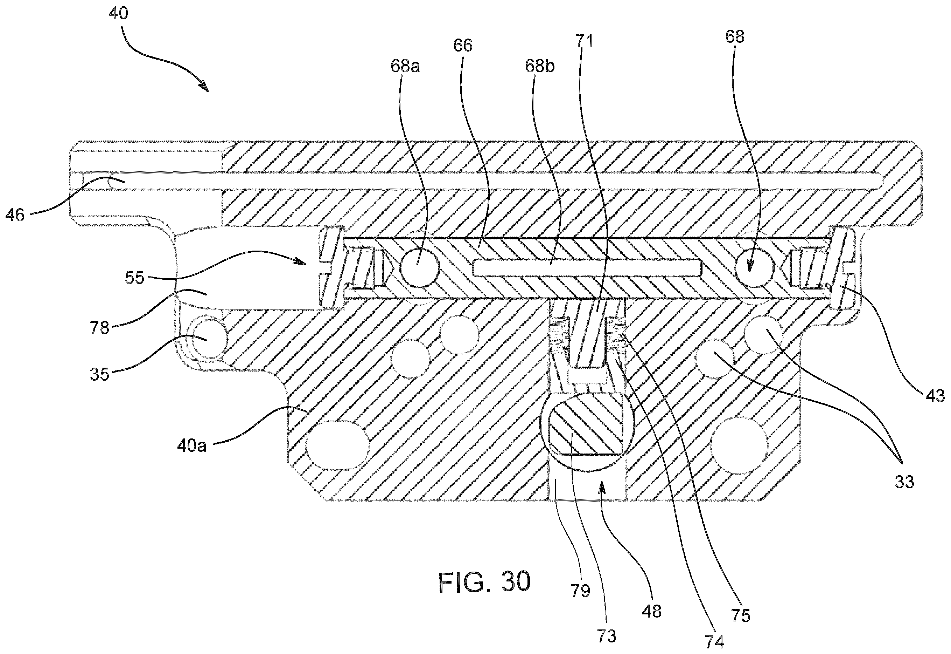

**FIG. 30** is an anterior cross-sectional view of the pivoting tibial resection guide according to the claimed invention.

### DETAILED DESCRIPTION OF THE PREFERRED EMBODIMENTS

The following detailed description of the preferred embodiments is presented only for illustrative and descriptive purposes and is not intended to be exhaustive or to limit the scope of the invention. The scope of the invention is only limited by the appended claims. The embodiments were selected and described to best explain the principles of the invention and its practical application.

Similar reference characters indicate corresponding parts throughout the several views unless otherwise stated. Although the drawings represent embodiments of various features and components according to the present disclosure, the drawings are not necessarily to scale and certain features may be exaggerated to better illustrate embodiments of the present disclosure, and such exemplifications are not to be construed as limiting the scope of the present disclosure.

Except as otherwise expressly stated herein, the following rules of interpretation apply to this specification: (a) all words used herein shall be construed to be of such gender or number (singular or plural) as such circumstances require; (b) the singular terms "a," "an," and "the," as used in the specification and the appended claims include plural references unless the context clearly dictates otherwise; (c) the antecedent term "about" applied to a recited range or value denotes an approximation with the deviation in the range or values known or expected in the art from the measurements; (d) the words, "herein," "hereby," "hereto," "hereinbefore," and "hereinafter," and words of similar import, refer to this specification in its entirety and not to any particular paragraph, claim, or other subdivision, unless otherwise specified; (e) descriptive headings are for convenience only and shall not control or affect the meaning of construction of part of the specification; and (f) "or" and "any" are not exclusive and "include" and "including" are not limiting. Further, the terms, "comprising," "having," "including," and "containing" are to be construed as open-ended terms (*i.e.,* meaning "including but not limited to").

References in the specification to "one embodiment," "an embodiment," "an exemplary embodiment," *etc.,* indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments, whether explicitly described.

Recitation of ranges of values herein are merely intended to serve as a shorthand method of referring individually to each separate value falling within the range of any sub-ranges there between, unless otherwise clearly indicated herein. Each separate value within a recited range is incorporated into the specification or claims as if each separate value were individually recited herein. Where a specific range of values is provided, it is understood that each intervening value, to the tenth or less of the unit of the lower limit between the upper and lower limit of that range and any other stated or intervening value in that stated range of sub range thereof, is included herein unless the context clearly dictates otherwise. All subranges are also included. The upper and lower limits of these smaller ranges are also included therein, subject to any specifically and expressly excluded limit in the stated range.

It should be noted that some of the terms used herein are relative terms. For example, the terms, "upper" and, "lower" are relative to each other in location, *i.e.,* an upper component is located at a higher elevation than a lower component in each orientation, but these terms can change if the orientation is flipped. The terms, "inlet" and "outlet" are relative to the fluid flowing through them with respect to a given structure, e.g., a fluid flows through the inlet into the structure and then flows through the outlet out of the structure. The terms, "upstream" and "downstream" are relative to the direction in which a fluid flows through various components prior to flowing through the downstream component.

The terms, "horizontal" and "vertical" are used to indicate direction relative to an absolute reference, *i.e.,* ground level. However, these terms should not be construed to require structure to be absolutely parallel or absolutely perpendicular to each other. For example, a first vertical structure and a second vertical structure are not necessarily parallel to each other. The terms, "top" and "bottom" or "base" are used to refer to locations or surfaces where the top is always higher than the bottom or base relative to an absolute reference, *i.e.,* the surface of the Earth. The terms, "upwards" and "downwards" are also relative to an absolute reference; an upwards flow is always against the gravity of the Earth.

There are many reasons that a patient may undergo a total knee arthroplasty ("TKA"). Such reasons may include trauma, the progression of a bone degenerative disease, and excessive wear due to time and robust use. Common bone degenerative diseases include rheumatoid arthritis and arthrosis.

In a primary TKA, (*i.e.,* a TKA in which the surgeon operates on a knee joint that has not previously been operated upon) the surgeon generally makes a vertical midline incision on the anterior side of the operative knee. The incision is generally made with the knee in flexion at or below the tibial tuberosity and may extend several inches above the patella. The surgeon then continues to incise the fatty tissue to expose the anterior aspect of the joint capsule. A medial parapatellar arthrotomy may be performed to pierce the joint capsule and resect the medial patellar retinaculum. A retractor is then commonly used to move the patella laterally to expose the distal condyles of the femur and the cartilaginous meniscus resting on the proximal tibial plateau. The surgeon then removes the meniscus and uses instrumentation to measure and resect the distal femur and proximal tibia. The resected distal femur and the resected proximal tibia will eventually accommodate the endoprosthetic knee implants.

The type of measurement and resection instrumentation used may be influenced by the surgeon's preference for a particular joint placement school of thought. These joint placement schools of thought can influence the designs of available knee endoprosthetic and their associated instrumentation. The three main knee joint placement philosophies are known respectively as anatomic alignment, mechanical alignment, and kinematic alignment.

The oldest alignment school of thought is the anatomic alignment philosophy. In anatomic alignment, the surgeon attempts to resect the tibia at three degrees of varus regardless of the orientation of the patient's actual pre-diseased joint line. Femoral resections and ligament releases are also performed to keep a straight hip-knee-ankle axis of the limb. Releasing the anterior cruciate ligament ("ACL") to accommodate the implant can lead to patient feelings of weakness as described further below. Additionally, implant technology of the time was not yet prepared to handle the effects of the three degree varus resection of the tibia. For example, the varus angle created shear forces between the tibial implant, the meniscal insert, and the femoral implant, which contributed to implant failure.

The angle of resection of the distal femur essentially sets the angle of the axis of the prosthetic joint. Anatomic alignment does not allow the angle of resection to float indefinitely. This can result in an angle of resection that does not align with the native angle of the patient's pre-diseased joint. As a result, anatomic alignment can lead to patient discomfort, weakening of the surrounding soft tissue (*e.g*., ligaments and muscle) and premature wear of the prosthetic.

In mechanical alignment, the surgeon resects the tibia perpendicular to the mechanical axis of the tibia. The mechanical axis of the tibia generally refers to an axial line extending from the center of rotation of a proximal head of the associated femur through the center of the knee to a center of the ankle. A perpendicular resection of the proximal aspect of the tibia relative to the mechanical axis results in a resection that is coplanar with a transverse plane disposed at the resection area. Many tibial prosthetics designed for mechanical alignment sit on the resected tibial plateau and have articular surfaces configured to place the new joint line parallel to the transverse plane of resection. That is, the reconstructed joint line is also perpendicular to the mechanical axis. Approaching the same concept form a different perspective, a mechanically reconstructed joint line can generally be visualized as being parallel to a flat floor when the knee is in extension and the patient is standing. By contrast, the location of the natural joint line varies from person to person, but on average, the natural joint line has a slight varus tilt relative to a transverse plane of the patient's body.

The mechanical alignment technique can provide good stability when the patient's leg is in extension (e.g., when the patient is standing), and sometimes this technique is required due to trauma or severe disease progression, but the implants that are commonly used with mechanical alignment often require the release of the ACL. In some circumstances, the posterior cruciate ligament ("PCL") may also be released. The ACL normally prevents the tibia from sliding too far anteriorly and from rotating too far relative to the femur. The absence of either of these ligaments can lead to feelings of weakness when the leg is in flexion. Furthermore, changing the location of the patient's natural joint line can lead to feelings of discomfort. Patients who alter their gait to accommodate the new joint line may chronically stress the remaining muscles, which can further exacerbate the feelings of discomfort and contribute to additional musculoskeletal problems in the future.

Resection of the ACL also encourages the use of a "gap balancing" technique in which the surgeon uses a distractor to apply an opposing force to the tibia and the femur in an attempt to set the distal cut surface of the femur parallel to the proximal cut surface of the tibia while symmetrically tensioning the surviving collateral ligaments. The surviving collateral ligaments are typically the lateral collateral ligament ("LCL"), which connects the femur to the fibula on the lateral side, and the medial collateral ligament ("MCL"), which connects the femur to the tibia on the medial side. It is thought that by setting the distal cut surface of the femur parallel to the resected tibial plateau while the distraction forces are evenly balanced on the surviving collateral ligaments, a prosthetic can easily be inserted into the gap disposed between the femur and the tibia. It is thought that the forces of the knee in flexion and extension can be evenly distributed through the prosthetic, thereby avoiding uneven wear and other complications.

However, the anterior profile of the gap is generally trapezoidal after the femoral distal cut has been made. Surgeons are generally taught to create a rectangular gap to accommodate the endoprosthetic implant. To do this, the surgeons commonly release the MCL if the knee is a varus knee, and the LCL if the knee is a valgus knee. Valgus knees are present in a smaller population of patients. The release of these ligaments creates scar tissue as the reattached ligaments begin to heal after a successful procedure. The healed ligament often undergoes contracture as a result of scarring. The ligament release also subjects the tissue to trauma, creates a source of additional bleeding, and can generally prolong patient recovery time.

The newest alignment school of thought is the kinematic alignment philosophy. The kinematic alignment philosophy recognizes that every patient's physiology is slightly different and seeks to restore the patient's natural pre-diseased joint line by taking actual measurements of the operative physiology to ascertain the position of the native joint line. While nothing precludes the use of the present claimed instrument in mechanical or anatomic alignment, the inventors recognized the shortcomings of mechanical and anatomic alignment and invented a device that is also compatible with kinematic alignment.

There are various ways to conduct a kinematic alignment procedure, but all start by referencing the distal condyles of the femur. Most methods involve the evaluation of the thickness of the articular cartilage on the distal aspects of the femoral condyles. Surgeons may use calipers, a cartilage thickness gauge such as the one disclosed in U.S. Pat. Pub. No. 2019/0231365, or other instruments to measure the amount of cartilage wear. The position of the native pre-diseased joint line is largely set by the interaction between the soft tissue (e.g., articular cartilage) on femoral condyles and the tibial plateau as supported by the underlying bone. In the absence of bone loss, knowing the thickness of the pre-diseased cartilage ultimately permits the measurement of the pre-diseased joint line. For example, if the surgeon measured 2 millimeters ("mm") of wear on the medial condyle and no wear on the lateral condyle, and if the surgeon plans to use a 10 mm endoprosthetic implant, the surgeon can set a femoral resection guide on the anterior surface of the femur for the purpose of performing the distal cut of the distal aspect of the femoral condyles. The femoral resection guide can be angled to resect 10 mm of the distal aspect of the lateral condyle and 8 mm of the distal aspect of the medial condyle. The 8 mm of resection on the medial condyle plus the 2 mm of measured cartilage loss will therefore accommodate the 10 mm implant on the medial side. Likewise, the 10 mm resection of the lateral condyle will accommodate the 10 mm implant on the lateral side.

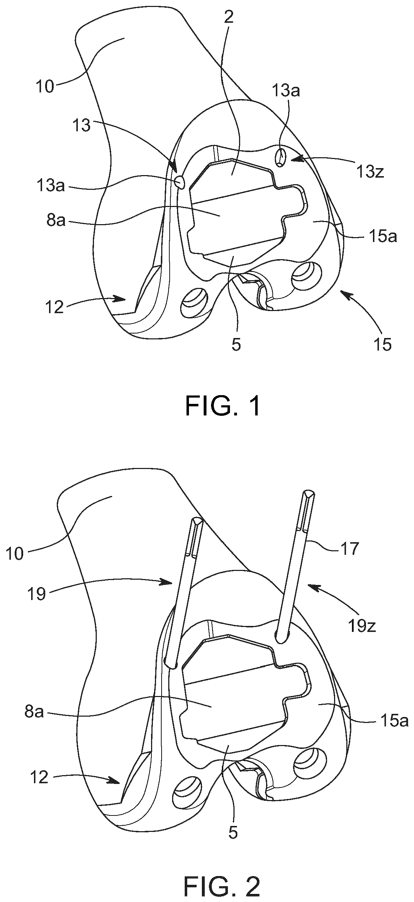

The surgeon then uses a sizing guide or a sizing caliper to size the implant. Surgical kits typically include several implant size options to accommodate variations in the patient population. Once the sizing guide has been used to inform the surgeon of the appropriate implant size, the surgeon then removes the sizing guide and places a four in one cutting block on the resected distal surface of the femur. The four in one cutting block has saw slots that permit the surgeon to make the anterior, posterior, and two chamfer cuts (see **FIG. 1****).**

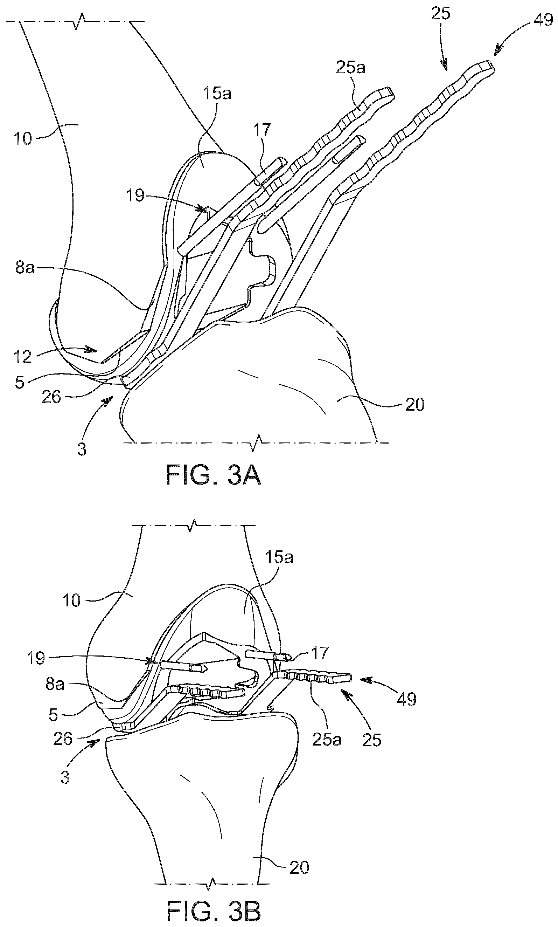

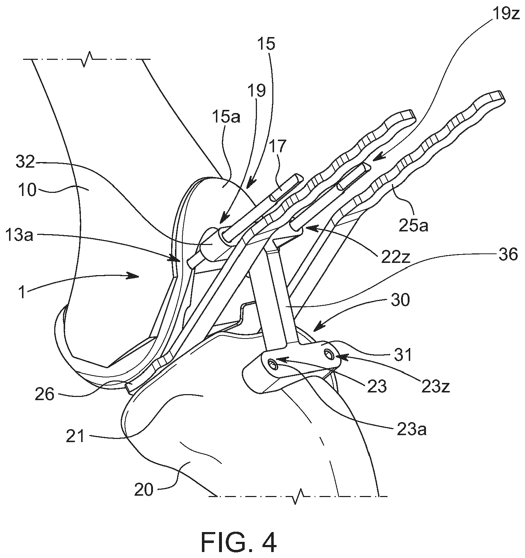

A femoral implant trial (see **15a)** is then placed on the resected distal end **12** of the femur **10.** The femoral implant trial **15a** desirably matches the sizing dimensions of the endoprosthetic implant that will be later installed. Spreading or traction devices (for example, gap spacers (see **25a,** **FIG. 4****))** are then inserted into the joint gap **3 (****FIG. 3A****)** to measure the medial and lateral dimensions of the joint gap **3.**

To determine the amount of proximal tibial resection, the measured dimensions of the medial and lateral aspects of the joint gap are subtracted from the desired thickness of resection. For example, if the surgeon plans to use a 10 mm tibial implant and the medial gap is 3 mm and the lateral gap is 1 mm, the surgeon will orient the tibial resection guide to resect 7 mm of the medial side of the tibial plateau and 9 mm of the lateral side of the tibial plateau. It will be appreciated that in a kinematic alignment technique, the release of the MCL or the LCL is typically unnecessary. If the distal cut surface is not initially parallel to the proximal tibial cut surface, the surgeon typically recuts the tibia until the surgeon has achieved the desirable rectangular-shaped joint gap.

Adjusting the position of the tibial resection guide based on the measurements of the spreading or traction devices creates an angle of resection that has been calculated based upon the patient's particular anatomy. Based upon these measurements, the tibial resection guide is typically oriented at a varus angle relative to the transverse plane, but for some patients, the angle may be valgus or close to 0°. Because of the geometry of the meniscal insert and the femoral component of the endoprosthetic implant assembly, the reconstructed joint line is generally parallel to the plane of tibial resection. Replicating the natural pre-diseased joint line is a significant step in restoring the balanced natural kinematics of the knee. A kinematically balanced knee avoids the problems of the mechanical and anatomic schools of thought.

Described herein are instruments, assemblies, kits, systems, and methods that may be configured to be used in primary total knee arthroplasties ("TKAs").

### Linking Drill Guide Technique with Engaging Femoral Trial

**FIGS. 1 - 10** generally depict method steps, exemplary devices, and assemblies that include an exemplary linking drill guide **30** and that can involve the use of a femoral trial having first and a second complementary femoral engagement member **13, 13z.**

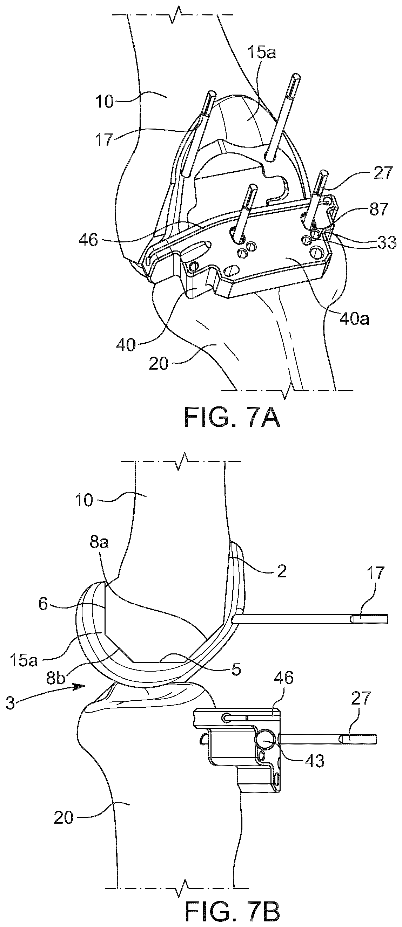

**FIG. 1** is a perspective view depicting a resected distal end **12** of a femur **10.** As shown in **FIG. 1****,** the femur **10** is prepared using a preferred resection technique. The distal, anterior/posterior and chamfer cuts are made to form the distal resected surface **5,** posterior resected surface **6,** anterior resected surface **2,** and chamfer resected surfaces **8a, 8b** respectively (see also **FIG. 7B****).** The chamfer resected surfaces **8a, 8b** comprise the anterior chamfer resected surface **8a** and the posterior chamfer resected surface **8b.** A four in one cutting block may desirably be used to create the posterior resected surface **6,** anterior resected surface **2,** and chamfer resected surfaces **8a, 8b,** but it will be understood that other instrumentation may be used in lieu of or in addition to the four in one cutting block per the surgeon's preference. A femoral referencing instrument **15** is provided. The depicted femoral referencing instrument **15** is a femoral trial **15a** having a first complementary femoral engagement member **13** and a second complementary femoral engagement member **13z** in the form of femoral reference holes **13a.** Trial implants, such as the depicted femoral trial **15a,** are test endoprostheses that generally have the same functional dimensions of the actual endoprostheses, but trial implants are designed to be temporarily installed and easily removed for the purposes of evaluating the fit of the actual endoprostheses and for the purposes of evaluating the knee joint's kinematics. The surgeon generally removes the trial implants and installs the actual implants once the surgeon is satisfied with the trial implant's sizing and the knee joint's kinematics.

In other exemplary embodiments, the femoral referencing instrument **15** can be a distal referencing guide **15b (****FIG. 12A****),** a femoral distal resection guide **15c (****FIG. 16****),** pins **15d (****FIG. 17A****)** or any other instrument disposed on, or associated closely with the distal end **12** of the preferably distally resected surface **5** of the operative femur **10** that can be used directly or indirectly to ascertain the position of the distal aspect of the resected femur **10** relative to the proximal aspect of the tibia **20** of the same leg and, when used in conjunction with an exemplary distally referencing linking drill guide assembly **1** in an engaged configuration, mechanically transfers information about the orientation of the distal resected surface **5** of the femur directly or indirectly to the tibia **20.** Stated differently, the femoral referencing instrument **15** can be used to mechanically transfer information about the orientation of the distal resected surface **5** of the femur directly to the tibia **20** (or indirectly through an intermediate instrument such as a pivoting tibial resection guide **40)** when the exemplary distally referencing linking drill guide assembly **1** is in the engaged configuration.

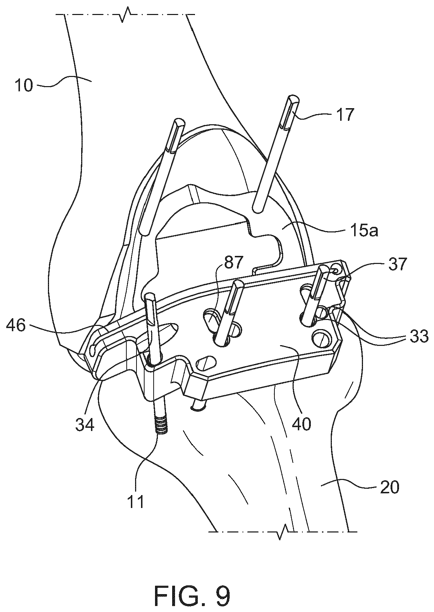

As shown in **FIG. 2****,** each of the femoral reference holes **13a** (that is, the example first and second complimentary femoral engagement members **13, 13z)** receive a femoral linking pin **17.** The leading ends (see the leading end **11** of the divergent fixation pin **34** in **FIG. 9** for reference) of these femoral linking pins **17** are in turn placed into the distal portion of the resected femur **10.** In the depicted embodiment, the femoral linking pins **17** function as first and second femoral engagement elements **19, 19z.**

It will be appreciated that the femoral linking pins **17** may be common pins, headless nails, drill bits, posts, or other connectable fasteners that are compatible with standard pin slots of associated instrumentation such as the distal femoral resection guide **15c (****FIG. 16****),** pivoting tibial resection guide **40** (FIG. **7A****),** the linking drill guide **30,** other drill guides, and other surgical instrumentation. It will also be appreciated that in other exemplary embodiments, the femoral linking pins **17** may have visual or tactile indicators to mitigate surgeon error. Visual indicators can include different colors and markings. Visual and tactile indicators can include raised or recessed portions of the femoral linking pin **17.**

Although the exemplary embodiments of **FIGS. 1 - 10** depict a first complementary femoral engagement member **13** and a second complementary femoral engagement member **13z** in the form of femoral reference holes **13a,** it will be understood that other exemplary embodiments may comprise one complementary femoral engagement member **13.** Still other exemplary embodiments may comprise more than two complementary femoral engagement members **13, 13z,** *etc.* The complementary femoral engagement member **13** may take the form of a hole, a slot, a recess, a protrusion, a clamp, a lip, a magnet, a spike, or any other structure known in the art used to selectively (whether directly or indirectly) fixedly engage and disengage one component to another component, and any combination thereof. It will be appreciated that in certain exemplary embodiments, the complementary femoral engagement member **13** may be present in the distal femur **10** itself and the femoral engagement member **19** of the linking drill guide **30** can be configured to engage the complementary femoral engagement member **13** disposed directly in the distal femur **10.** In such an exemplary embodiment, the complementary femoral engagement member **13** is likely to be a bore hole disposed directly in the distal femur **10** made by a drill bit guided by a drill guide.

As shown in **FIG. 2****,** femoral linking pins **17** are drilled through the femoral reference holes **13a** in the femoral trial **15a.** In certain exemplary embodiments, the femoral linking pins **17** may be threaded. In such embodiments, the femoral linking pins **17** are desirably threaded at a leading end (see **FIG. 9****)** to fixedly engage the femoral linking pin **17** into the bone. The femoral linking pins **17** are left in place in the femoral trial **15a.**

As shown in **FIG. 3A** and **FIG. 3B****,** spreading devices **49** in the form of gap spacers **25** are inserted medially and laterally to fill the joint gap **3** defined by the area between the distal aspect of the femur **10** and the proximal aspect of the tibia **20.** It will be understood that gap spacer **25** can refer to any instrument that can be inserted into the space between the proximal aspect of the tibia and the distal aspect of the femur **10** that is used to measure or otherwise evaluate the size of the joint gap **3** (*i.e.,* the height of the joint gap **3** medially and laterally). In this manner, such spreading devices are "configured to be disposed" between a resected distal femur **10** and a proximal tibia **20** to ascertain a distance between the distal femur **10** and the proximal tibia **20.** It will be appreciated that the spreading devices **49** can be disposed in the joint gap **3** when the knee is in flexion or extension.

In the embodiment depicted in **FIGS. 3A** and **3B****,** the gap spacer **25** is a spoon gap spacer **25a.** The measurement element **26** of the spoon gap spacer **25a** is desirably curved to closely abut the curved surface of the femoral trial **15a.** It will be understood that many gap spacers **25** having differently dimensioned measurement elements **26** are typically provided for a procedure. The differently sized measurement elements **26** are typically provided in 1 mm height increments, but other increments are contemplated. **FIG. 3B** shows the measurement element **26** of the medial gap spacer **25** (*i.e.,* the gap spacer **25** depicted on the left side of the image) being thicker than the measurement element **26** of the adjacent lateral gap spacer **25** (*i.e.,* the gap spacer **25a** depicted on the right side of the image).

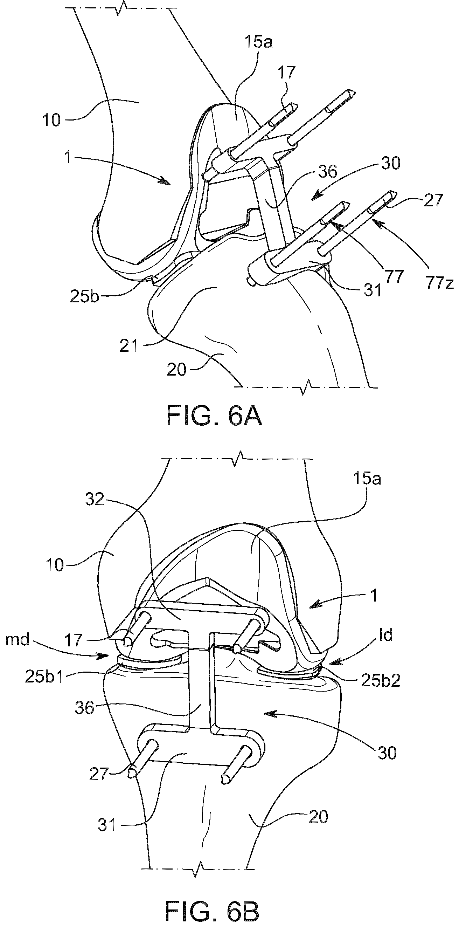

Spoon gap spacers **25a** may be provided for the left and right leg. Different embodiments of gap spacers **25** include the snap-on spacers **25b** shown in **FIG. 6A****,** differently dimensioned trays or plugs configured to be inserted into the joint space **3,** and spacers with removable measurement elements **26.** Nothing in this disclosure limits the types of gap spacers **25** that are compatible with the exemplary embodiments of this disclosure.

Furthermore, it will be appreciated that spreading devices **49** can include gap spacers **25,** lamina spreaders, a ratcheting tensioner, or other ligament tensioning devices such as gap balancing devices. In certain exemplary methods, traction devices may be used in lieu of or in addition to spreading devices. Whereas spreading devices are typically inserted into the operative area of the knee to separate the distal femur **10** from the proximal tibia **20** at the joint space **3,** a tensioning device is typically disposed outside of the operative area, such as on the patient's ankle or leg to pull on the operative leg to thereby provide traction and separate the distal femur **10** from the proximal tibia **20** at the joint space **3.** It will be appreciated that a tensioning device can include a boot configured to enclose a portion of the patient's leg, ankle, or foot on the operative leg, a surgeon's or technician's hands, or other device configured to apply traction to the operative leg.

After the distal cut of the femur has been made, the surgeon will selectively insert differently sized spoon gap spacers **25a** into the medial and lateral sides of joint gap **3** until the measurement end of the spoon gap spacers **25a** provide a secure fit. Without being bound by theory, it is contemplated that the use of a spreading device **49** (such as for example, the spoon gap spacers **25a)** in combination with the use of the linking drill guide **30** in the engaged position as described further below, can obviate the need for "gap balancing" and the release of either the MCL or the LCL and thereby avoid the risk of hematoma, unnecessary trauma to the ligaments, scarring, and increased healing time that would otherwise result in a traditional mechanical alignment technique. The spreading device **49** effectively sets the joint gap **3** at the desired distance while the linking drill guide **30** transfers information about the orientation of the distal femoral cut to the tibial resection guide (which can be a pivoting tibial resection guide) to permit the surgeon to quickly make a tibial cut that is usually desirably parallel to the distal femoral cut. The distal femoral cut and the proximal tibial cut effectively define the "rectangle" into which the endoprosthetic implant will be inserted.

Previously, in a mechanical alignment technique, the MCL or the LCL would be released (*i.e.,* cut) to define the "rectangle" into which the endoprosthetic implant would be inserted. In this manner, it is contemplated that the linking drill guide **30** can used to avoid the need for "gap balancing," the release of the MCL or the LCL, and the associated scaring, hematoma, trauma, and increased healing time of traditional methods. These improvements can be especially pronounced when an exemplary linking drill guide **30** is used in a mechanical alignment technique.

It is contemplated that the exemplary embodiments described herein can be used with the mechanical alignment technique, anatomic alignment technique, kinematic alignment technique, or any other technique practiced by a qualified orthopedic surgeon.

**FIG. 4** shows the exemplary distally referencing linking drill guide assembly **1** in the engaged configuration. Exemplary distally referencing linking drill guide assemblies **1** generally comprise a femoral referencing instrument **15** and a linking drill guide **30.**

An exemplary linking drill guide **30** has a femoral portion **32,** the femoral portion **32** being configured to engage a first femoral engagement member **19.** The first femoral engagement member **19** is in turn configured to engage the first complementary femoral engagement member **13** of the femoral referencing instrument **15.** The depicted linking drill guide **30** is further configured to engage a second femoral engagement member **19z.** The second femoral engagement member **19z** is configured to engage the second complementary femoral engagement member **13z (****FIG. 1****)** of the femoral referencing instrument **15.** The linking drill guide **30** may be referred to as a "yoke" if desired.

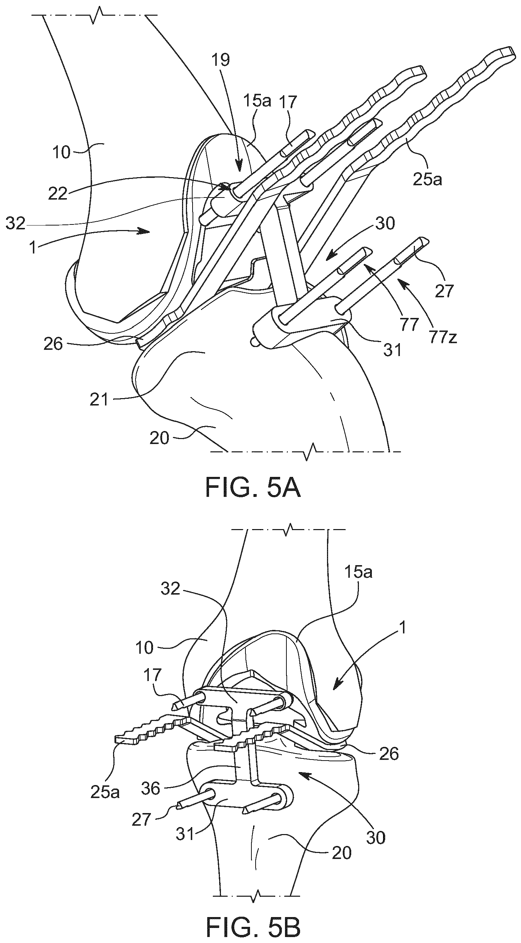

The femoral portion **32** of the linking drill guide **30** has areas defining femoral linking holes **22 (****FIG. 5A****), 22z.** A femoral linking hole **22** is an example structure that permits the femoral portion **32** of the linking drill guide **30** to engage the first femoral engagement member **19.** By having a femoral linking hole **22** that can be disposed around the femoral linking pin **17,** the linking drill guide **30** can be said to "indirectly engage" the femoral referencing instrument **15** via a first femoral engagement member **19,** (which in the depicted embodiment takes the form of the femoral linking pin **17)** and first complementary femoral engagement member **13,** (which in the depicted embodiment takes the form of a first femoral reference hole **13a (****FIG. 1****)).** In such an exemplary manner, the femoral portion **32** can thereby be said to be, "configured to engage a first femoral engagement member **19."**

Likewise, the second femoral linking hole **22z** is an example structure that permits the femoral portion **32** to engage a second femoral engagement member **19z.** By having a second femoral linking hole **22z** that can be disposed around the second femoral linking pin **17,** the linking drill guide **30** can be said to "indirectly engage" the femoral referencing instrument **15** via a second femoral engagement member **19z,** (which in the depicted embodiment takes the form of the femoral linking pin **17)** and second complementary femoral engagement member **13,** (which in the depicted embodiment takes the form of a second femoral reference hole **13z).** In such an exemplary manner, the femoral portion **32** can thereby be said to be, "configured to engage a second femoral engagement member **19z."**

While a femoral linking pin **17** and a femoral linking hole **22** are provided as an example for what may be provided for the femoral portion **32** to be configured to engage a femoral engagement member **19, 19z,** *etc.,* it will be appreciated that any mechanical engagement mechanism designed to selectively engage one component to another is considered to be within the scope of this disclosure. Furthermore, while first and second femoral reference holes **13a** are provided as an example of a first complementary femoral engagement member **13** and a second complementary femoral engagement member **13z** that receive the distal end of the femoral linking pins **17** (*e.g.,* femoral engagement members **19, 19z,** *etc*.) and that are thereby "configured to engage" the femoral engagement members, it will be appreciated that any mechanical engagement mechanism designed to selectively engage one component to another is considered to be within the scope of this disclosure.

It will be also appreciated that in other exemplary embodiments, the first femoral engagement member **19** is an integral part of the linking drill guide **30.** For example, the first femoral engagement member **19** may be permanently engaged to the femoral portion **32** and can extend directly from the femoral portion **32** of the linking drill guide **30** (see **FIGS. 13A** and the blade **19c** in **FIG. 17B****).** In embodiments where the linking drill guide **30** comprises the first femoral engagement member **19** that is permanently engaged to the femoral portion **32,** such embodiments can likewise be said to be, "configured to engage a first femoral engagement member **19."** The same can be said for exemplary linking drill guides **30** that comprise a second or even more than two permanently engaged femoral engagement members **19z,** *etc.* A linking drill guide **30** that comprises a permanently affixed femoral engagement member **19, 19z,** *etc.* can further be said to "directly engage" the femoral referencing instrument **15** via a first femoral engagement member **19,** and a first complementary femoral engagement member **13.**

Likewise, in embodiments where the linking drill guide **30** comprises a second femoral engagement member **19z,** such embodiments can likewise be said to be, "configured to engage a second femoral engagement member **19."** A linking drill guide **30** that comprises a second femoral engagement member **19z** can further be said to "directly engage" the femoral referencing instrument **15** via a second femoral engagement member **19z,** and a second complementary femoral engagement member **13z.**

In the depicted embodiment, the first and second femoral engagement members **19, 19z** are femoral linking pins **17, 17z** and the first and second complementary femoral engagement members **13, 13z** of the distal femoral referencing instrument **15** are femoral reference holes **13a.**

It will be appreciated that in other exemplary embodiments, the first or second femoral engagement member **19, 19z** can comprise a slot, a lip, a clamp, hook, protrusion, recesses, spike, magnet, an orientation pin **19b (****FIG. 13B****),** a blade **19c (****FIG. 17B****),** or any other structure known in the art used to directly or indirectly selectively fixedly engage and disengage one component to another component and any combination thereof. In certain exemplary embodiments, the first or second femoral engagement member **19, 19z** physically contacts the first complementary femoral engagement member **13** without an intermediary element. In such embodiments, the first or second femoral engagement member **19, 19z** can be said to "directly engage" the first or second complementary femoral engagement member **13, 13z.** Likewise if an intermediary element is present, the first or second femoral engagement member **19, 19z** can be said to "indirectly engage" the first or second complementary femoral engagement member **13, 13z.**

The linking drill guide **30** further comprises a tibial portion **31.** The tibial portion **31** has areas defining two tibial reference holes **23.** It will be appreciated that in certain exemplary embodiments, only one tibial reference hole **23** may be provided. In yet other exemplary embodiments, more than two tibial reference holes **23, 23z** may be provided. A body **36** connects the femoral portion **32** to the tibial portion **31.** The body **36** of the linking drill guide **30** and the generally parallel disposition of the reference indicia (*e.g.,* the femoral linking holes **22, 22z** and the tibial reference holes **23, 23z)** on the respective femoral portion **32** and the tibial portion **31,** transfers the information regarding the orientation of the plane of distal resection (which is coplanar with the distal resected surface **5)** to the tibial portion **31** of the linking drill guide **30.** In certain exemplary embodiments, the body **36** may have a fixed length. Multiple linking drill guides **30** each comprising a body **36** that has a length that is different from another body **36** of another linking drill guide **30** provided in a kit may be provided. In such exemplary embodiments, the surgeon may select one linking drill guide **30** of the multiple provided linking drill guides **30** to transfer the information about the distal resected surface **5** of the femur **10** to a tibial resection guide **40 (****FIG. 7A****)** for the purpose of setting the plane of tibial resection. In certain procedures, the plane of tibial resection is desirably parallel to the plane of distal resection.

In other exemplary embodiments, the body **36** can have an adjustable length dimension relative to the femoral portion **32,** tibial portion **31,** or both the femoral portion **32** and the tibial portion **31.** In yet other exemplary embodiments, a length of the femoral portion 32 or the tibial portion **31** can be adjustable relative to the body **36.** In any embodiment comprising an adjustment of the length of the linking drill guide **30,** the adjustable components are desirably able to be locked at a desired length. It is contemplated that kits that feature such adjustable length linking drill guides may contain fewer linking drill guides **30** than kits that contain multiple linking drill guides 30 having multiple different lengths.

In certain exemplary embodiments, the linking drill guide **30** is provided as a unitary piece. It is contemplated that a unitary piece can be easier to sterilize between procedures and may obviate the risk of mechanical failure compared to exemplary embodiments in which the linking drill guide **30** is not a unitary piece, but rather comprises two or more components. The linking drill guide **30** is desirably sized to be placed anteriorly on the knee exposed in the surgical area. It is contemplated that the exemplary linking drill guides **30** described herein can reduce the overall instrumentation required to perform a TKA, while permitting the surgeon to resect the proximal tibia more quickly than what is safely achievable with existing instrumentation.

Similarly to the femoral linking holes **22, 22z** provided in the femoral portion **32,** the first tibial reference hole **23** of the tibial portion **31** permits the tibial portion **31** to engage a first tibial engagement member **77 (****FIG. 5A****).** The depicted embodiment comprises first and second tibial engagement members **77, 77z** in the form of the tibial linking pins **27.** By having a first tibial reference hole **23** that can be disposed around a tibial linking pin **27,** the linking drill guide **30** can be said to be, "configured to engage a first tibial engagement member **77."**

Likewise, the second tibial reference hole 23z is an example structure that permits the tibial portion 31 to engage a second tibial engagement member 17z. By having a second tibial reference hole 23z that can be disposed around the second tibial engagement member 77z, the tibial portion 31 can thereby be said to be, "configured to engage a second tibial engagement member 77z."

In the depicted embodiment, the first and second tibial engagement members 77, 77z are tibial linking pins 27, 27z.

It will be appreciated that in other exemplary embodiments, the first or second tibial engagement member 77, 77z can comprise a slot, a lip, a clamp, hook, protrusion, recesses, spike, magnet, an orientation pin, a blade, or any other structure known in the art used to directly or indirectly selectively fixedly engage and disengage one component to another component and any combination thereof. In certain exemplary embodiments, the first tibial engagement member **77** physically contacts the tibia **20** without an intermediary element. In such embodiments, the first tibial engagement member **77** can be said to "directly engage" the tibia **20.** It is contemplated that in certain exemplary embodiments, an intermediate element may be disposed between the tibial portion **31** and the tibia **20.** In such embodiments, the first or second tibial engagement member **77, 77z** may engage the intermediate component and the intermediate component may itself directly engage the tibia **20.** In such embodiments, the first or second tibial engagement member **77, 77z** can be said to "indirectly engage" the tibia **20.**

It is contemplated that exemplary linking drill guides **30** in accordance with this disclosure can be manufactured from (or coated with) any clinically proven biocompatible material, including stainless steel, cobalt chromium alloys, or a plastic polymer such as ultra-high molecular weight polyethylene ("UHWPE"). In certain exemplary embodiments, the linking drill guides **30** can be single-use disposable linking drill guides. In other exemplary embodiments, the linking drill guides **30** can be designed for use in multiple surgical procedures. Regardless of embodiment, the exemplary linking drill guide **30** is desirably sterilized prior to entering the surgical field.

As shown in **FIG. 4****,** a linking drill guide **30** is slid over the femoral linking pins **17** until a tibial portion **31** of the linking drill guide **30** contacts the anterior tibial cortex **21** of the proximal tibia **20.** The sliding of the femoral linking holes **22** of the femoral portion **32** over the linking pins **17** extending through the femoral reference holes **13a** of the femoral trial **15a** in the depicted embodiment defines the engaged configuration of the distally referencing linking drill guide assembly **1.** Likewise, it will be appreciated that the distally referencing linking drill guide assembly **1** is in a disengaged configuration when the femoral engagement member **19** is not directly or indirectly engaged to a complementary femoral engagement member **13.** In the depicted embodiment for example, the distally referencing linking drill guide assembly **1** is in the disengaged configuration when the femoral linking holes **22** of the femoral portion **32** are not disposed around the linking pins **17.**

While femoral linking holes **22** are provided by way of example, it will be appreciated that the femoral linking holes **22** may take the form of a slot, a recess, a tube, a protrusion, a clamp, a lip, a magnet, a spike, or any other structure known in the art used to selectively (whether directly or indirectly) fixedly engage and disengage one component to another component, and any combination thereof. It will also be appreciated that in embodiments wherein the femoral engagement member or members **19, 19z,** *etc.* are integrally engaged to the linking drill guide **30** (see for example, **FIGS. 13B** and **17B****),** the femoral linking holes **22** can be absent.

Spreading devices **49** such as gap spacers **25,** may be in place prior to sliding the linking drill guide **30** over the femoral linking pins **17,** or the spreading devices **49** may be placed in the joint gap **3** after the linking drill guide **30** has been slid over the femoral linking pins **17.**

As shown in **FIG. 5A** and **FIG. 5B****,** with the spoon gap spacers **25a** in place, tibial linking pins **27** are placed in each of the tibial reference holes **23, 23z,** *etc.* in the linking drill guide **30.** In the depicted embodiment, the tibial reference holes **23, 23z.** are tibial drill holes **23a.** In other exemplary embodiments, a tibial reference hole **23** can take the form of a hole, a slot, a tube, a recess, a protrusion, a clamp, a lip, a magnet, a spike, or any other structure known in the art used to selectively (whether directly or indirectly) fixedly engage and disengage one component to another component, and any combination thereof. It will also be appreciated that in embodiments wherein the tibial engagement member or members are integrally engaged to the linking drill guide **30,** the tibial reference holes **23, 23z** can be absent. In certain exemplary embodiments, only one tibial reference hole **23** may be present. The tibial linking pins **27** are secured to the tibia **20.** In certain exemplary embodiments, the tibial linking pins **27** are threaded. In such exemplary embodiments, the tibial linking pins **27** are desirably threaded at a leading end **11** (see the leading end **11** of the divergent fixation pin **34** in **FIG. 9** for reference) to fixedly engage the tibial linking pin **27** into the tibia **20.**

Alternatively, as shown in **FIGS. 6A** and **6B****,** snap-on spacers **25b** can be used in place of the gap spoons **25a** to fill the joint gap **3.** It will be appreciated that the measurement element **26** of the snap-on spacers **25b** can occupy substantially the same area as the snap-op spacer **25b** itself. **FIG. 6B** provides an illustrative example of the medial snap-on spacer **25b1** being thicker than the adjacently disposed lateral snap-on spacer **25b2.**

As shown in **FIG. 7A****,** the linking drill guide **30** is removed, leaving the femoral linking pins **17** in place in the femur **10** and the tibial linking pins **27** in place in the tibia **20.** The receiving slots **87** of a pivoting tibial resection guide **40** are slid over the tibial linking pins **27.** In the depicted embodiment, the receiving slots **87** extend generally anteriorly-posteriorly through the body **40a** of the pivoting tibial resection guide **40.** The receiving slots **87** are desirably sized to closely encompass the medial-lateral width of the tibial linking pins **27** while having a generally vertical (*i.e.,* superior to inferior) length dimension that permits the pivoting tibial resection guide **40** to pivot around the tibial linking pins **27** as described further below.

**FIG. 30** is a cross-sectional anterior view of a pivoting tibial resection guide **40** according to the claimed invention. The pivoting tibial resection guide **40** can comprise a pivoting tibial resection guide body **40a.** The body **40a** defines a generally linear resection slot **46** disposed above a pivoting recess **78.** A pivoting assembly **55** can be closely dimensioned to rotate axially within the pivoting recess **78.** The pivoting assembly **55** itself is disposed above a locking mechanism recess **79** that is dimensioned to closely enclose a locking mechanism **48.** The body **40a** further has areas defining multiple standard holes **33,** +2mm standard pin holes **33,** and a divergent fixation pin receiving hole **35.**

The depicted locking mechanism **48** comprises a cam **73,** a cam follower **74,** a shaft **71** substantially perpendicularly oriented to a pivoting guide **66** and springs **75** disposed between the cam follower **74** and the shaft **71.** The pivoting assembly **55** comprises a pivoting guide **66** and end screws **43** placed on either end of the pivoting guide **66** prevent the pivoting guide **66** from sliding out of the pivoting tibial resection guide **40.** The pivoting guide **66** desirably has one or more complimentary tibial engagement members **68** that can receive a tibial engagement member associated with the drill linking guide **30.** Complimentary tibial engagement members **68** may include a slot **68b** dimensioned to receive the linking tab **64** of the spike plate **67** (see the embodiment depicted in **FIG. 20B)** and tibial engagement holes **68a** disposed adjacent to either lateral side of the slot **68b.** However, in certain exemplary embodiments, either one or more tibial engagement holes **68a** may be provided in lieu of the slot **68b** and vice versa.

As shown in the side view of **FIG. 7B****,** the tibial resection level is set automatically to accommodate a 10 mm tibial component (or construct) of an endoprosthetic assembly, which can comprise a tibial trial base **93 (****FIG. 29****)** and a meniscal trial insert **95 (****FIG. 29****).** In embodiments, the linking drill guide **30** can be made to allow for other resection levels as needed or desired by the surgeon, such as by a plurality of linking drill guides **30,** multiple pin holes on the linking drill guide **30,** multiple linking holes on the pivoting tibial resection guide **40,** or an adjustable linking drill guide **30.**

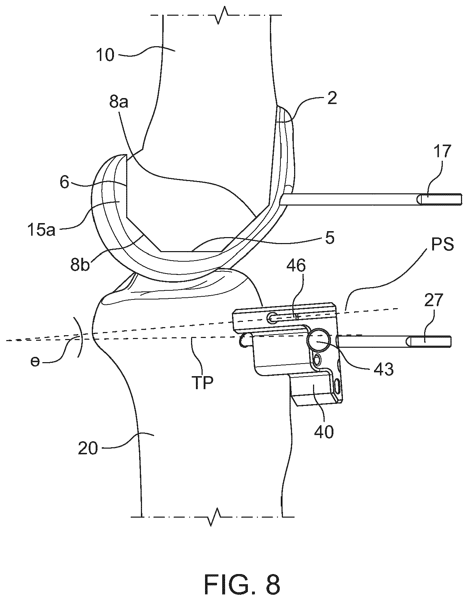

As shown in **FIG. 8****,** the tibial posterior slope **PS** can be adjusted as needed to match the natural anatomy. The pivoting tibial resection guide **40** is configured such that the pivoting tibial resection guide **40** can be adjusted relative to the placement of the tibial linking pins **27** in the tibia **20.** The tibial posterior slope **PS** can be imagined as a plane extending generally anteriorly-posteriorly and medially and lateral that passes through the rection slot **46.** In **FIG. 8****,** the side view of this plane is depicted as a line. The intersection of the tibial posterior slope **PS** and a transverse plane **TP** extending generally horizontally through the tibia **20** defines the posterior slope angle **θ**. The placement of the transverse plane **TP** can be measured from any frame of reference that is useful to define the posterior slope angle **θ**. In the depicted embodiment, the transvers plane **TP** is disposed coplanar with the tibial linking pins **27** extending into the tibia **20.** In the side view of **FIG. 8****,** the transverse plane **TP** is represented as a line. In embodiments, the pivoting tibial resection guide **40** can be adjusted from about minus 3 degrees to about plus 10 degrees relative to the tibial linking pins **27.** Once the desired tibial posterior slope **PS** is reached, the pivoting tibial resection guide **40** can be locked in place using a locking mechanism **48** on the pivoting tibial resection guide **40.**

As shown in **FIG. 9****,** once the posterior slope **PS** is set at the desired posterior slope angle **θ**, the pivoting tibial resection guide **40** is pinned in place by inserting pins **37** through standard pin holes **33** and into the tibia **20.** The standard pins holes **33** depicted include the 10 mm pin holes disposed below and slightly offset from the +2 mm standard pin holes **33.** The pins **37** extend through the standard pin holes **33** in **FIG. 9****.** The + 2mm standard pin holes **33** can be used if the sizing guide indicates that that patient's anatomy would require a tibial construct (*i.e.,* meniscal trial insert 95, and tibial trial base **93,** see **FIG. 29****)** greater than the standard 10 mm. In common practice however, the pivoting tibial resection guide **40** is more likely to be replaced back onto the pins **37** in the +2 mm standard pin holes **33** if the amount of tibial resection was insufficient to allow for a 10 mm tibial construct. It will be appreciated that other exemplary pivoting tibial resection guides **40** can have more than four pin holes **33.** All practicable increments between standard pin holes **33** are considered to be within the scope of this disclosure.

In **FIG. 9****,** the tibial linking pins **27** have been removed from the receiving slots **87** of the pivoting tibial resection guide **40.** The pins **37** extend through the standard pin holes **33** into the tibia **20** generally prohibit further pivoting. Rather, these pins **37** can be used to fix the pivoting tibial resection guide **40** in the desired orientation. As shown, a divergent fixation pin **34** can be used if desired for added stability. The leading end **11** of the divergent fixation pin **34** extends into the anterior tibial cortex **21** of the proximal tibia **20.**

As illustrated with reference to **FIG. 9****,** other resection levels of the proximal tibia **20** can also be realized with a plurality of standard pin holes **33** disposed at different resection levels. Sliding the standard pin holes **33** disposed at different elevations of the pivoting tibial resection guide **40** over resection levels changes the position of the resection slot **46** superiorly and inferiority relative to the top of the tibial plateau and thereby permits the surgeon to adjust the amount of resection to accommodate endoprosthetic implant assemblies of different sizes.

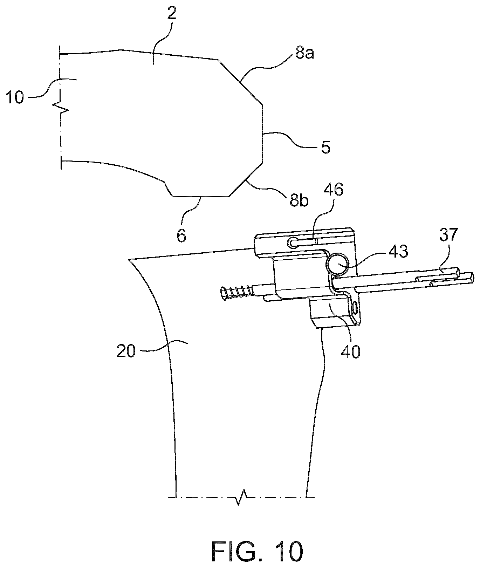

As shown in **FIG. 10****,** the femoral trial **15a** is removed and a proximal tibial resection is performed through the resection slot **46** in the pivoting tibial resection guide **40.** The surgeon typically uses a handheld surgical saw inserted through the resection slot **46** to make the resection. In other exemplary embodiments, the surgeon can use the top of the pivoting tibial resection guide **40** as a plane upon which to align the proximal tibial resection.

Once the tibial resection is complete, the surgeon selects an appropriate size tibial trial base **93** and meniscal trial insert **95.** The femoral trial **15a** is then re-placed on the resected distal end **12** of the femur **10** and a trial reduction is carried out. The femoral sulcus, peg prep, and tibial keel prep can be performed according to a kinematic alignment technique or surgeon preference.

### Distal Referencing Technique

**FIGS. 11 - 15** generally depict method steps and exemplary devices and assemblies that include another exemplary linking drill guide **30,** wherein the femoral referencing instrument **15** is a distal referencing guide **15b** having complementary femoral engagement members **13.**

**FIG. 11** is a perspective view of a knee placed in extension, wherein the distal femur **10** has been resected and spoon gap spacers **25a** have been inserted medially and laterally to fill a joint space between the resected femur **10** and the intact tibia **20** to determine the medial and lateral gap distance. The surgeon or technician will then select snap-on spacers **25b** out of many available snap-on spacers **25b,** wherein a first snap-on spacer has a thickness that is different from another available snap-on spacer **25b.** Once selected thicknesses snap on spacers **25b** of an appropriate thickness are selected, the fully assembled distal referencing guide **15b** matches the gap distance **d** determined in the step using the spoon gap spacers **25a** (see **FIG. 11****).**

In certain exemplary methods, the use of the spoon gap spacers **25a** can be omitted, and the distal referencing guide **15b** with snap-on gap spacers **25b** that are selectively chosen to match the distance of the medial and lateral dimension of the joint gap **3** can be used in lieu of the spoon gap spacers **25a.**

In certain exemplary methods, the pivoting tibial resection guide **40** can also be used in a distal referencing technique. At the beginning of the distal referencing technique, the distal end **12** of the femur **10** is resected. The distal, anterior, posterior, and chamfer cuts are made to form the distal resected surface **5,** posterior resected surface **6,** anterior resected surface **2,** and chamfer resected surfaces **8a, 8b** respectively (see also **FIG. 14B****).** U.S. Pat. App. Pub. No. 2019/0231365A1, provides one example of how a surgeon may orient the distal, anterior, posterior, and chamfer cuts. As shown in **FIG. 12B****,** after the distal resection, the knee is placed in extension and distal referencing guide **15b** is inserted into the joint space **3.** In the depicted embodiment, the distal referencing guide **15b** comprises a distal referencing portion **44,** a modular handle **41,** and snap-on gap spacers **25b** disposed on a bottom side of the distal referencing portion **44.**

As seen in **FIG. 12A****,** the snap-on gap spacers **25b** are affixed to the distal referencing portion **44** of the distal referencing guide **15b.** The surgeon uses the modular handle **41** to insert the referencing portion **44** medially and laterally between the distal aspect of the resected femur **10** and the proximal aspect of the tibia **20** to fill the joint space **3** and determine the medial gap distance **md (****FIG. 12B****)** and the lateral gap distance **ld (****FIG. 13B****).** Unlike in the technique described above, a femoral trial **15a** is not placed on the resected femur **10** during the linking transfer steps.

The distal referencing technique is carried out using a distal referencing guide **15b.** An exemplary embodiment of an assembled distal referencing guide **15b** is shown in **FIG. 12A****.** The distal referencing guide **15b** has a femoral referencing portion **44** having a medial partial condylar portion **45M** and a lateral partial condylar portion **45L.** Each condylar portion **45M, 45L** has a complementary femoral engagement member **13.** In the depicted exemplary embodiment, the complementary femoral engagement member **13** is a pin hole bore **13b** formed anterior-to-posterior therethrough. Each pin hole bore **13b** can be accessed anteriorly for use in orienting a linking drill guide **30,** as indicated in **FIG. 13A****.** A modular handle **41** is configured to readily attach and detach from the femoral referencing portion **44** for use in maneuvering the distal referencing guide **15b** into and within the joint space **3.** A distal portion of each of the condylar portions **45M, 45L** is configured to receive a snap on spacer **25b.** A plurality of thicknesses of snap on spacers **25b** are provided. Appropriate thicknesses of snap on spacers **25b** are selected such that the fully assembled distal referencing guide **15b** matches the gap distance **d** determined in the prior step using the spoon gap spacers **25a** (see **FIG. 11****).**

**FIG. 12B** shows use of the modular handle **41** to slide the distal referencing guide **15b** into the joint space **3.** As shown in **FIG. 12C****,** the modular handle **41** can be removed from the distal referencing guide **15b** after insertion, if desired.

As shown in **FIG. 13A****,** with the distal referencing guide **15b** in place in the joint space **3,** a linking drill guide **30** is inserted into the incision. The depicted exemplary embodiment of the linking drill guide **30** has a recess **39** in the femoral portion **32** of the linking drill guide **30** to permit the modular handle **41** to be inserted through the recess **39** to engage the distal referencing portion **44** of the distal referencing guide **15b** if desired. A pair of femoral engagement members **19, 19z** in the form of orientation pins **19b** are formed on or adjacent the femoral portion **32** of the linking drill guide **30.** It will be appreciated that in other exemplary embodiments, femoral linking holes **22, 22z,** or other femoral engagement members **19** may be used in lieu of or in addition to the orientation pins **19b** depicted in **FIG. 13A****.** The orientation pins **19b** are inserted into the pin hole bores **13b** in the condylar portions **45M, 45L** until the tibial portion **31** of the linking drill guide **30** contacts the anterior tibial cortex **21.** In this manner, the exemplary distally referencing linking drill guide assembly **1** is disposed in an engaged configuration. The linking drill guide **30** includes a pair of tibial reference holes **23, 23z** on or adjacent the tibial portion **31** of the linking drill guide **30.**

A modified embodiment of the linking drill guide **30** and associated assembly, systems, and methods described with reference to **FIGS. 13A** is further contemplated. In such an embodiment, the orientation pins **19b** of the linking drill guide **30** can be inserted directly into drill bores made in the distal femur **10.** The exemplary linking drill guide **30** can be sized to have the orientation pins **19b** extend into drill bore formed via a distal femoral resection guide (see **15c,** **FIG. 16****).** In certain exemplary embodiments, the orientation pins **19b** can be spikes configured to engage the drill bores in the distal femur **10.** In such exemplary embodiments, the spikes are the femoral engagement members **19** and the drill bores in the distal femur **10** are the complementary femoral engagement members **13.**

As shown in **FIG. 13B****,** tibial linking pins **27** are placed in each of the tibial reference holes **23, 23z** in the linking drill guide **30.** As shown in **FIG. 13C****,** the modular handle **41** can optionally be used in this step for stability. The tibial linking pins **27** are inserted into the tibia **20.** In certain embodiments, the tibial linking pins **27** are threaded at the leading end **11** (see **FIG. 15****).**

As indicated in **FIG. 14A****,** the linking drill guide **30** and the distal reference guide **15b** is removed from the joint, leaving the tibial linking pins **27** in place in the proximal tibia **20.** The receiving slots **87** of a pivoting tibial resection guide **40** are slid onto the tibial linking pins **27.** From this point on, the technique is similar to the technique described above, except that a femoral trial **15a** is not on the resected distal femur **10.** It should be noted that once the linking pins **27** are placed into the tibia **20,** there is no need to the femoral trial **15a** to remain in place for any of the embodiments described herein.

As indicated in the side view of **FIG. 14B****,** the instruments can be sized such that the tibial resection level is set automatically to match a 10 mm tibial implant construct (*i*.*e.,* a tibial trial base **93** plus a meniscal trial insert **95).** In embodiments, the linking drill guide **30** can be made to allow for other resection levels as needed or desired by the surgeon, such as by a plurality of linking guides, multiple pin holes, or an adjustable guide.

As shown in **FIG. 14C** and with reference to **FIG. 8****,** the tibial posterior slope **PS** can be adjusted as needed to match the natural anatomy, in the manner described above. The pivoting tibial resection guide **40** is configured such that the pivoting tibial resection guide **40** can be adjusted relative to the placement of the tibial linking pins **27.** In embodiments, the pivoting tibial resection guide **40** can have a posterior slope angle **θ** in the range of about minus 3 degrees to about plus 10 degrees relative to the linking pins **27.** Once the posterior slope angle **θ** is set, the pivoting tibial resection guide **40** can be locked at the selected posterior slope angle **θ**.

As described above with reference **FIG. 9****,** the pivoting tibial resection guide **40** is pinned in place through the standard pin holes **33.** A divergent fixation pin **34** can be used if desired for added stability. The tibial linking pins **27** are removed.

As shown in **FIG. 15****,** a proximal tibial resection is performed through the resection slot **46** in the pivoting tibial resection guide **40.** Unlike in the technique described above, there is no femoral trial **15a** to remove prior to carrying out the tibial resection.

Once the tibial resection is complete, the surgeon selects an appropriate size tibial base and tibial insert trials. A femoral trial **15a** is placed on the previously resected distal femur **10.** If the distal referencing technique as described herein is used, the femoral trial **15a** may not have a complementary femoral engagement member **13** such as the complementary femoral engagement member **13** disclosed in **FIGS. 1** - **9****.** A trial reduction is carried out (see **FIG. 29****).** The femoral sulcus, peg prep, and tibial keel prep can be performed according to the kinematic alignment technique or surgeon preference.

### Distal Femoral Resection Guide Referencing Technique

**FIGS. 16 - 28** generally depict method steps and exemplary devices and assemblies that include other embodiments of exemplary linking drill guide **30** and that can involve the use of a distal femoral resection guide **15c.**

**FIG. 16** is a perspective view of a knee in flexion. In the embodiments depicted in these figures, the femoral referencing instrument **15** is a distal femoral resection guide **15c.** The distal femoral resection guide **15c** has been slid over femoral linking pins **17** extending into the femur **10.** In this embodiment, the femoral linking pins **27** are the standard pins that are otherwise used to locate the femoral distal cut guide. The distal femoral resection guide **15c** was placed using kinematic alignment techniques known to surgeons. For example, the distal femoral resection guide assembly disclosed in U.S. Pat. App. Pub. No. 2019/0231365A1 may have been used to orient the distal femoral resection guide **15c.** Once oriented, the surgeon locks the distal femoral resection guide's position and orientation relative to the distal femur **10** using the femoral linking pins **17.** The surgeon then inserts a surgical saw through the resection slot **57** to resect the distal aspect of the femur **10** to create the distal resected surface **5** via the distal cut.

With the exemplary linking drill guide **30** depicted in **FIG. 17A****,** the distal femoral resection guide **15c** is then removed and the leg is placed in extension. It will be appreciated that the exemplary embodiment of the linking drill guide **30** used can influence which instrument serves as the femoral referencing instrument **15.** For example, in **FIG. 17A****,** the femoral resection guide **15c** has been removed and the femoral linking pins **17, 15d** remain disposed in the femur **10.** Because the femoral linking pins **17** together with the depicted embodiment of the linking drill guide **30** can be used to mechanically transfer information about the orientation of the distal resected surface **5** of the femur **10** to the tibial resection guide **40,** the femoral linking pins **17** in the depicted embodiment serve as a type of femoral referencing instrument **15.** Similarly, in **FIG. 17B****,** wherein the slot **57** of the distal femoral resection guide **15c,** when engaged to the blade **19c** of the exemplary linking drill guide **30,** mechanically transfers information about the orientation of the orientation of the distal resected surface **5** of the femur **10** to the tibial resection guide **40,** the distal femoral resection guide **15c** is a type of femoral referencing instrument **15.**

The exemplary linking drill guide **30** shown in **FIG. 17A** comprises a femoral portion **32.** The femoral portion **32** comprises tubes **24** that can receive first and second femoral engagement members **19, 19z** in the form of femoral linking pins **17.** That is, the tubes **24** define linking holes **22** (see **FIG. 5A****).** The tubes **24** and linking holes **22** can receive the femoral linking pins **17**in much the same manner as described above with reference to **FIGS. 4** and **5A****.** In this manner, the depicted embodiment is configured to engage a first and a second femoral engagement member **19, 19z.** In the depicted embodiment, the first and second complementary femoral engagement members **13, 13z** are drill bores made directly into the distal femur **10.** In **FIG. 17A****,** the femoral linking pins **17**also serve as the first and second femoral engagement members **19, 19z.** The distal femoral resection guide **15c** has been removed and the placement of the femoral linking pins **17**in the distal aspect of the femur **10** retain the information about the plane of distal resection, which is coplanar with the distal resected surface **5.** That is, an imaginary shortest possible line connecting the two femoral linking pins **17**can function as a reference line that is parallel to the plane of distal resection of the femur **10.**

The exemplary linking drill guide **30** further comprises a tibial portion **31** having tubes **24** defining tibial reference holes **23, 23z,** and a body **36** connecting the femoral portion **32** to the tibial portion **31.** A handle **38** may optionally be provided to facilitate installation and removal of the linking drill guide **30.** The handle **38** may be removable, or the handle **38** may be a permanent and integral part of the linking drill guide **30.** The body **36** of the linking drill guide **30** transfers the information regarding the orientation of the plane of distal resection (which is coplanar with the distal resected surface **5)** to the tibial portion **31** of the linking drill guide **30.**

As better seen in **FIGS. 18****,** **19A,** and **19B****,** the distal end **16** of the tubes **24** define the tibial reference holes **23, 23z.** The distal end **16** of each of the tubes **24** is preferably recessed from the posterior distal end **18** of the tibial portion **31** of the linking drill guide **30.** The inferior surface **53 (****FIG. 19B****)** of the tibial portion **31** aligns with the tibial resection plane when the linking drill guide **30** is in the engaged configuration. This feature permits the surgeon to visualize the tibial resection cut before it is made. The recess **39** further permits direct marking of the tibia **20** by creating a line connecting the two opposing inferior surfaces **53** at the distal end **18** of the tibial portion **31.** Direct marking is commonly performed with surgical grade single use marker or through a cautery device. Depending upon preference, a line drawn through the recess **39** separating the opposing inferior surfaces **53** at the distal end **18** of the tibial portion **31** may be made.

**FIG. 18** further shows spreading devices **49** such as gap spacers **25** inserted into the joint gap **3** to ascertain and verify the medial and lateral distance between the resected distal condyles of the femur **10** and the medial and lateral condyles of the adjacent proximate tibial plateau **20** of the same leg. Lamina spreaders or other tensioning devices may be used to apply tension to the joint in place of the distal referencing gap spacers **25.** Once determined, the surgeon inserts the tibial linking pins **27** as depicted in **FIG. 19A****.**

**FIG. 20A** shows the tibial linking pins **27** remaining disposed in the proximal aspect of the anterior cortex **21** of the tibia **20** after the linking drill guide **30** has been removed. As seen in **FIG. 21A** and **22****,** the receiving slots **87** of a pivoting tibial resection guide **40** can then be slid over the remaining tibial linking pins **27.** The locking mechanism **48** is in an unlocked position. The anterior face of the locking mechanism **48** can be provided with a visual indicator to let the surgeon know which position the locking mechanism **48** is in. In the depicted embodiment, the indicator disposed at the 9 o'clock position indicates that the locking mechanism **48** is unlocked. The posterior slope **PS** (see **FIG. 8****)** can then be adjusted and the proximal aspect of the tibia **20** can be resected as described above (see also generally **FIGS. 23** - **28****).**

Referring to **FIG. 17B****,** another exemplary embodiment of a linking drill guide 30 is provided. In the depicted embodiment of the associated distally referencing linking drill guide assembly **1,** the distal femoral resection guide **15c** can be viewed as serving as the femoral referencing instrument **15.** The femoral resection slot **57** also functions as the first complementary femoral engagement member **13** of the femoral referencing instrument **15.** The first femoral engagement member **19** on the femoral portion **32** of the linking drill guide **30** is a blade **19c** that is dimensioned to fit closely into the femoral resection slot **57.** In the depicted embodiment, a recess **39** may be present in the tibial portion **31** of the linking drill guide **30.** The recess **39** permits the linking drill guide **30** to be slid over a stem portion **51** of the distal femoral resection guide **15c.**

The blade **19c** of the linking drill guide **30** is slid into the femoral resection slot **57** of the distal femoral resection guide **15c** with the leg in extension. **FIG. 19B** shows the tibial linking pins **27** having been inserted into the tibial reference holes **23.** It is then possible to proceed as outlined in **FIGS. 21A** and **22** and as described above.

**FIG. 20B** shows a spike plate **67** being used as a reference instrument for the pivoting tibial resection guide **40** instead of tibial linking pins **27.** As shown in **FIGS. 19A** and **19B****,** the tibial linking pins **27** are inserted through the tibial reference holes **23, 23z** in the tibial portion **31** of the linking drill guide **30** and are drilled into the anterior cortex **21** of the proximal tibia **20.** In **FIG. 20B,** both the linking drill guide **30** and the tibial linking pins **27** have been removed, thereby leaving the drill bores in the anterior cortex **21** of the proximal tibia **20.** A spike plate **67** comprising a first spike member **61** and second spike member **62** connected by a body portion **63** and having a linking tab **64** to facilitate selective engagement to the pivoting tibial resection guide **40** is provided. As discussed further below, the linking tab **64** can be representative of the orientation of the plane otherwise formed by the compound axes of the tibial linking pins **27.** The first and second spoke members **61, 62** of the spike plate **67** are inserted into the tibial drill bore holes left by the linking tibial pins **27.**

In certain exemplary embodiments, the spike plate **67** may be a single use, disposable item. In other exemplary embodiments, the spike plate may be made from stainless steel or any other clinically proven biocompatible material of sufficient strength and durability.

As seen in **FIG. 21B****,** a pivoting tibial resection guide **40** having a reference slot **41** is slid over the linking tab **64** of the spike plate **67.** The linking tab **64** is visible through the open resection slot **41.** The linking tab **64** is oriented parallel to the resection slot **46** and is thereby indicative of the orientation of the plane otherwise formed by the compound axes of the tibial linking pins **27** (see **PS,** **FIG. 8****).** The linking tab **64** may optionally be provided with a visual indicator such as a different color from the surrounding instrumentation to better facilitate the surgeon's previsualization of the tibial rection cut, which is made on a parallel plane above the plane otherwise formed by the compound axes of the tibial linking pins **27.** The surgeon can lock the linking tab **64** to the reference slot **41** using the locking mechanism **48** on the anterior end of the pivoting tibial resection guide **40.** In **FIG. 21B****,** the locking mechanism **48** is shown in the unlocked position.

The spike plate **67** permits medial and lateral positioning of the pivoting tibial resection guide **40** as well as internal and external rotation of the pivoting tibial resection guide **40,** whereas the use of the tibial linking pins **27** precludes medial and lateral positioning of the pivoting tibial resection guide **40.**

**FIGS. 23 - 25** depict a tibial visual slope gage **60** that may be optionally placed into the resection slot **47** of the pivoting tibial resection guide **40.** The posterior slope angle **θ** of the tibial resection guide **40** can be adjusted from about -3° to about +10° relative to the placement of the tibial linking pins **27,** or relative to the spike plate **67** placement in embodiments involving the use of the spike plate **67.** Once the desired slope and orientation is achieved, the pivoting action may be locked by rotating the locking mechanism **48.**

The locking mechanism **48** depicted in **FIG. 21B** and in the cross-sectional view of **FIG. 30** is in an unlocked position. The locking mechanism **48** may be a friction locking mechanism such as the one depicted in **FIG. 30****,** but other locking mechanism configured to selectively fix the orientation of the pivoting tibial resection guide **40** are considered to be within the scope of this disclosure. For example, a mechanical locking mechanism **48** may be provided in certain exemplary embodiments.

With reference to the locking mechanism of **FIG. 30** and with further reference to **FIG. 27****,** a surgeon or technician can lock the locking mechanism by inserting a keyed instrument through the interface of the locking mechanism **48.** The keyed instrument may be a screwdriver, hex key, or other keyed instrument having a keyed end of any shape that is dimensioned to closely engage a complementary key shape in an interface that communicates rotationally with the cam **73.** Upon rotating the keyed instrument, the interface rotates the cam **73** in the same direction. The cam **73** translates the rotational force to linear force by pushing the cam follower **74** against the springs **75.** The springs **75** in turn transfer the linear force to the shaft **71** and the shaft **71** in turn transfers the linear force as friction to the pivoting guide **66.** The application of this friction thereby prevents the pivoting assembly **55** from rotating axially. In this manner, the surgeon or technician can be said to "lock" the pivoting tibial resection guide at the desired posterior slope angle **θ**.

It will be understood that in embodiments comprising tibial linking pins **27,** the tibial linking pins **27** extend through the tibial engagement holes **68a** of the pivoting guide **66** to selectively engage the pivoting tibial resection guide **40** to the tibia **20.** The tibial engagement holes **68a** generally align with the receiving slots **87** of the pivoting tibial resection guide **40.** Likewise, in embodiments that include a linking tab **64,** the slot **68b** of the pivoting guide **66** is desirably dimensioned to receive the linking tab **64** of the spike plate **67.**

As shown in **FIG. 26****,** the slope gage is removed after the slope of the pivoting tibial resection guide **40** is set. Standard pins **37** are then placed through the standard pin holes **33** in the pivoting tibial resection guide **40.** The pivoting tibial resection guide **40** may be moved from the standard holes to the +2 mm location if desired. The locking mechanism **48** is shown in the locked position. In the depicted embodiment, the visual indicator in the 12 o'clock position indicates that the locking mechanism **48** is locked. Other visual indicators that indicate the position of the locking mechanism are considered to be within the scope of this disclosure.

**FIG. 27** shows the pivoting tibial resection guide **40** disposed at the desired posterior slope **PS** (see **FIG. 8****)** and further depicts a divergent fixation pin **34** extending through the pivoting tibial resection guide **40** to further secure the pivoting tibial resection guide **40** to the tibia **20** at the desired slope. The resection slot **46** in the pivoting tibial resection guide **40** orients the resection plane of the proximal end **29** of the proximal tibia **20.**

**FIG. 28** depicts the pivoting tibial resection guide **40** disposed at the desired posterior slope **PS** (see **FIG. 8****),** wherein the proximal end **29 (****FIG. 27****)** of the proximal tibia **20** has been resected.

**FIG. 29** is a perspective view of a femoral trial **15a,** tibial trial base **93** and meniscal trial insert **95** that have been selected based on sizing criteria. Without being bound by theory, it is contemplated that the exemplary distally referencing linking drill guides **30** and/or the exemplary distally referencing linking drill guide assemblies **1** described herein can directly link the orientation of the distal cut (which results in the distal resected surface **5** of the distal femur **10)** to the orientation of the proximal cut of the proximal tibia **20** to thereby reduce the possibility for surgeon error significantly while further eliminating one or more extra steps otherwise required by prior independently referencing or indirectly linking kinematic alignment techniques. The minimal size of the exemplary distally referencing linking drill guides **30** and the exemplary distally referencing linking drill guide assemblies 1 allows the linking drill guide **30** and drill guide assembly 1 to fit in the operative area without the need for additional instrumentation that extends significantly outside of the incision. It is contemplated that the minimal amount of instrumentation may facilitate instrument re-sterilization between procedures.

The instruments can be provided in the form of a kit. The components of the kit are preferably arranged in a convenient format, such as in a surgical tray or case. However, the kit components do not have to be packaged or delivered together, provided that they are assembled or collected together in the operating room for use at the time of surgery. An exemplary kit can include any suitable embodiment of a linking drill guide **30,** variations of the linking drill guide **30** described herein, and any other linking drill guides **30** according to an embodiment. While it is contemplated that an exemplary kit may further include one or more femoral engagement members **19, 19z,** *etc.* one or more tibial engagement members **77, 77z,** *etc.,* and one or more femoral referencing instruments **15,** it will be appreciated that certain kits may lack some or all of these elements. Any suitable embodiment of a femoral engagement members **19,** variations of the femoral engagement members **19** described herein, and any other femoral engagement members **19** according to an embodiment are considered to be within the scope of this disclosure. Any suitable embodiment of a tibial engagement members **77,** variations of the tibial engagement members 77 described herein, and any other tibial engagement members **77** according to an embodiment are considered to be within the scope of this disclosure. Any suitable embodiment of a femoral referencing instrument **15,** variations of the femoral referencing instruments **15** described herein, and any other femoral referencing instrument **15** according to an embodiment are considered to be within the scope of this disclosure.

Selection of a suitable number or type of linking drill guide **30,** femoral engagement member **19,** tibial engagement member **77,** and femoral referencing instruments **15,** to include in a kit according to a particular embodiment can be based on various considerations, such as the procedure intended to be performed using the components included in the kit.

An exemplary medical device can comprise: a femoral portion, the femoral portion configured to engage a first femoral engagement member; a tibial portion, the tibial portion configured to engage a first tibial engagement member; and a body connecting the femoral portion to the tibial portion.

With such an exemplary medical device, the first femoral engagement member can be selected from the group consisting essentially of: a femoral linking pin, a blade, a slot, a lip, a clamp, a hook, a protrusion, a recesses, a spike, a magnet, an orientation pin, and combinations thereof.

With such an exemplary medical device, the first tibial engagement member can be selected from the group consisted essentially of: a hole, a slot, a recess, a protrusion, a clamp, a lip, a magnet, a spike, and combinations thereof.

An exemplary distally referencing linking drill guide assembly can comprise: a linking drill guide comprising: a femoral portion, the femoral portion configured to engage a first femoral engagement member, a tibial portion, the tibial portion configured to engage a first tibial engagement member, and a body connecting the femoral portion to the tibial portion; and a femoral referencing instrument, the femoral referencing instrument having a first complimentary femoral engagement member, the first complimentary femoral engagement member being configured to engage the first femoral engagement member, wherein the distally referencing linking drill guide assembly has an engaged configuration when the first femoral engagement member engages the first complimentary engagement member, and wherein the distally referencing linking drill guide assembly has a disengaged configuration when the first femoral engagement member does not engage the first complementary femoral engagement member.

Such an exemplary assembly may further comprise a spreading device configured to be disposed between a resected distal femur and a proximal tibia to ascertain a distance between the distal femur and the proximal tibia. The spreading device is selected from the group consisting essentially of a gap spacer, a lamina spreader, a ratcheting tensioner, or other knee ligament tensioning devices.

Such an exemplary assembly may have the femoral portion further configured to engage a second femoral engagement member and the femoral referencing instrument can further comprise a second complementary femoral engagement member, the second complementary femoral engagement member being configured to engage the second femoral engagement member. The second femoral engagement member can be configured to selectively engage the second complementary femoral engagement member.

Such an exemplary assembly may have a femoral referencing instrument that is selected from the group consisting essentially of: a femoral trial, a distal referencing guide, pins, and a femoral distal cut guide.

Such an exemplary assembly may have a tibial portion of the linking drill guide that is further configured to engage a second tibial engagement member.

Such an exemplary assembly may have an engaged configuration that comprises the first femoral engagement member directly engaging the first complimentary femoral engagement member.

Such an exemplary assembly may have an engaged configuration that comprises the first femoral engagement member indirectly engaging the first complimentary femoral engagement member.

Such an exemplary assembly may have a first femoral engagement member that is selected from the group consisting essentially of: a femoral linking pin, a blade, a slot, a lip, a clamp, a hook, a protrusion, a recesses, a spike, a magnet, an orientation pin, and combinations thereof.

Such an exemplary assembly may have a first tibial engagement member that is selected from the group consisted essentially of: a hole, a slot, a recess, a protrusion, a clamp, a lip, a magnet, a spike, and combinations thereof.

An exemplary medical device can comprise: a femoral portion, the femoral portion comprising a blade dimensioned to fit closely into a femoral resection slot of a distal femoral resection guide; a tibial portion, the tibial portion having tubes defining tibial reference holes; and a body connecting the femoral portion to the tibial portion.

Such an exemplary assembly may further comprise a handle engaged to the body.

Such an exemplary assembly may have tubes defining tibial reference holes that are recessed from a posterior distal end of the tibial portion of the linking drill guide.

Such an exemplary assembly may have an inferior surface of the tibial portion that aligns with a tibial resection plane when the linking drill guide is in the engaged configuration.

Another exemplary medical device can comprise: a femoral portion, the femoral portion comprising a first femoral engagement member; a tibial portion, the tibial portion configured to engage a first tibial engagement member; and a body connecting the femoral portion to the tibial portion.

Yet another medical device can comprise: a femoral portion, the femoral portion comprising a first femoral engagement member; a tibial portion, the tibial portion comprising a first tibial engagement member; and a body connecting the femoral portion to the tibial portion.

An exemplary method can comprise: engaging a femoral referencing instrument to a distal femur; engaging a first femoral engagement member directly or indirectly to the distal femur; and engaging a linking drill guide directly or indirectly to the first femoral engagement member, wherein the linking drill guide comprises: a femoral portion, the femoral portion configured to engage the first femoral engagement member, a tibial portion, the tibial portion configured to engage a first tibial engagement member, and a body connecting the femoral portion to the tibial portion.

In the exemplary method, the step of directly engaging the first femoral engagement member to the distal femur can comprise having the first femoral engagement member physically contact an anterior cortex of the distal femur.

In the exemplary method, the step of indirectly engaging the first femoral engagement member to the distal femur can comprise having the first femoral engagement member physically contact a first complementary engagement member on an intermediate femoral device, wherein a portion of the intermediate femoral device physically contacts the distal femur.

In the exemplary method, the step of indirectly engaging the first femoral engagement member to the distal femur can further comprise having the first femoral engagement member physically contact a first complementary engagement member on an intermediate device. A portion of the intermediate device can physically contact a subsequent intermediate device, and a portion of the subsequent intermediate device can physically contact the distal femur.

The exemplary method may further comprise inserting a spreading device into a joint space defined by the distal femur and a proximal femur of an operative leg to ascertain a distance between the distal femur and the proximal tibia. The distance comprises a medial distance and a lateral distance.

The exemplary method may further comprise using a traction device to pull the operative leg to apply traction to the proximal tibia or distal femur to expand the joint space.

The exemplary method may further comprise engaging a first tibial engagement member directly or indirectly to both the proximal tibia and the tibial portion of the linking drill guide.

In the exemplary method, the step of directly engaging the first tibial engagement member to the proximal tibia can comprise having the first tibial engagement member physically contact an anterior cortex of the proximal tibia.

In the exemplary method, the step of indirectly engaging the first tibial engagement member to the proximal tibia can comprise having the first tibial engagement member physically contact an intermediate tibial device. A portion of the intermediate tibial device can physically contact the proximal tibia.

In the exemplary method, the step of indirectly engaging the first tibial engagement member to the proximal tibia further can comprise having the first tibial engagement member physically contact an intermediate tibial device. A portion of the intermediate tibial device can physically contact a subsequent intermediate tibial device, and a portion of the subsequent intermediate tibial device can physically contact the proximal tibia.

The exemplary method may further comprise removing the linking drill guide from the first tibial engagement member, while leaving the first tibial engagement member directly or indirectly engaged to the proximal tibia.

The exemplary method may further comprise engaging a tibial resection guide to the first tibial engagement member.

The exemplary method may further comprise adjusting the posterior slope angle of the tibial resection guide relative to the position of the first tibial engagement member to define a desired resection slope.

The exemplary method may further comprise fixedly locking the tibial resection guide to the proximal tibia at the desired resection slope.

The exemplary method may further comprise resecting a tibial plateau of the proximal tibia at the desired resection slope.

The exemplary method may further comprise adjusting a height of the tibial resection guide relative to a top of the proximal tibia to accommodate a height of an endoprosthetic implant assembly.

The exemplary method may further comprise adjusting an internal/external angle of the tibial resection guide relative to the first tibial engagement member to define a desired internal/external resection angle.

The exemplary method may further comprise fixedly locking the tibial resection guide to the proximal tibia at the desired internal/external resection angle.

The exemplary method may further comprise resecting a tibial plateau of the proximal tibia at the desired internal/external resection angle.

The exemplary method may further comprise adjusting a medial/lateral position of the tibial resection guide relative to the first tibial engagement member to define a desired medial/lateral resection location.

The exemplary method may further comprise fixedly locking the tibial resection guide to the proximal tibia at the desired medial/lateral resection location.

The exemplary method may further comprise resecting a tibial plateau of the proximal tibia at the desired medial/lateral resection location.

It is to be understood that the present invention is by no means limited to the particular constructions and method steps herein disclosed or shown in the drawings, but the claimed invention is defined by the scope of the claims.

## Claims

1. A tibial resection guide (40) comprising:
a body (40a) defining a pivoting recess (78) extending laterally into the body (40a), and a generally linear resection slot (46) disposed above the pivoting recess (78);
multiple pin holes (33) extending anteroposteriorly through the body (40a); and
a pivoting assembly (55) disposed within the pivoting recess (78), the pivoting assembly (55) comprising one or more complimentary tibial engagement members (68, 68a, 68b),
wherein the pivoting assembly (55) is closely dimensioned to rotate axially within the pivoting recess (78) relative to the body (40a),
**characterized in that** the pivoting assembly (55) is disposed above a locking mechanism recess (79) of the body (40a) that is dimensioned to closely enclose a locking mechanism (48) comprised in the tibial guide (40),
the complimentary tibial engagement members (68, 68a, 68b) comprise
(i) a slot (68b) dimensioned to receive a linking tab (64) of a spiked plate (67) fixed on an anterior portion of a tibia,
(ii) and/or tibial engagement holes (68a) dimensioned to receive tibial linking pins (27) fixed on an anterior portion of the tibia, and
the locking mechanism (48) can be locked to prevent the pivoting assembly (55) from rotating axially.

2. The tibial resection guide (40) of claim 1, wherein axial rotation within the pivoting recess (78) permits the body (40a) to be rotated relative to the pivoting assembly (55) to define the desired posterior slope angle (θ) in a range of minus three degrees to plus 10 degrees relative to the tibial linking pins (27).

3. The tibial resection guide (40) of any of the previous claims, wherein the locking mechanism (48) has a locked configuration and an unlocked configuration, and wherein the locking mechanism (48) is configured to lock the tibial resection guide (40) at a desired posterior slope angle (Θ) in the locked configuration.

4. The tibial resection guide (40) of claim 3, wherein the locking mechanism (48) comprises: a cam (73), an interface, the interface rotationally communicating with the cam (73), a cam follower (74) linearly communicating with the cam (73), the cam follower (74) having a cam end and an opposing end distal to the cam end, a spring (75) disposed on the opposing end of the cam follower (74), the spring (75) having a cam follower end and a shaft end, and a shaft disposed at the shaft end.

5. The tibial resection guide (40) of claim 4, wherein the interface is a keyed interface.

6. The tibial resection guide (40) of any of the previous claims, wherein the locking mechanism (48) further comprises a visual indicator to indicate a position of the locking mechanism (48).

7. The tibial resection guide (40) of any of the previous claims, wherein the multiple pin holes (33) further comprise +2mm pin holes (33) and standard pin holes (33).

8. The tibial resection guide (40) of any of claims 1 to 7, wherein the multiple pin holes (33) further comprise a divergent fixation pin receiving hole (35).

## Patentansprüche

1. Tibiaresektionsführung (40), umfassend:
einen Körper (40a), der eine sich seitlich in den Körper (40a) hinein erstreckende Schwenkausnehmung (78) und einen oberhalb der Schwenkausnehmung (78) angeordneten, im Allgemeinen linearen Resektionsschlitz (46) definiert;
mehrere Stiftlöcher (33), die sich anteroposterior durch den Körper (40a) erstrecken; und
eine Schwenkbaugruppe (55), die innerhalb der Schwenkausnehmung (78) angeordnet ist, wobei die Schwenkbaugruppe (55) ein oder mehrere komplementäre tibiale Eingriffselemente (68, 68a, 68b) umfasst,
wobei die Schwenkbaugruppe (55) eng bemessen ist, um sich innerhalb der Schwenkausnehmung (78) relativ zu dem Körper (40a) axial zu drehen,
**dadurch gekennzeichnet, dass** die Schwenkbaugruppe (55) oberhalb einer Verriegelungsmechanismus-Ausnehmung (79) des Körpers (40a) angeordnet ist, die bemessen ist, um einen in der Tibiaführung (40) enthaltenen Verriegelungsmechanismus (48) eng zu umschließen,
die komplementären tibialen Eingriffselemente (68, 68a, 68b) umfassen
(i) einen Schlitz (68b), der bemessen ist, um eine Verbindungslasche (64) einer Stachelplatte (67) aufzunehmen, die an einem vorderen Abschnitt einer Tibia befestigt ist,
(ii) und/oder tibiale Eingriffslöcher (68a), die bemessen sind, um tibiale Verbindungsstifte (27) aufzunehmen, die an einem vorderen Abschnitt der Tibia befestigt sind, und
der Verriegelungsmechanismus (48) verriegelt werden kann, um zu verhindern, dass sich die Schwenkbaugruppe (55) axial dreht.

2. Tibiaresektionsführung (40) nach Anspruch 1, wobei die axiale Drehung innerhalb der Schwenkausnehmung (78) es ermöglicht, dass der Körper (40a) relativ zu der Schwenkbaugruppe (55) gedreht wird, um den gewünschten hinteren Neigungswinkel (θ) in einem Bereich von minus drei Grad bis plus 10 Grad relativ zu den tibialen Verbindungsstiften (27) zu definieren.

3. Tibiaresektionsführung (40) nach einem der vorhergehenden Ansprüche, wobei der Verriegelungsmechanismus (48) eine verriegelte Konfiguration und eine entriegelte Konfiguration aufweist, und wobei der Verriegelungsmechanismus (48) dazu konfiguriert ist, die Tibiaresektionsführung (40) in der verriegelten Konfiguration bei einem gewünschten hinteren Neigungswinkel (θ) zu verriegeln.

4. Tibiaresektionsführung (40) nach Anspruch 3, wobei der Verriegelungsmechanismus (48) umfasst: einen Nocken (73), eine Schnittstelle, wobei die Schnittstelle drehend mit dem Nocken (73) in Verbindung steht, einen Nockenfolger (74), der linear mit dem Nocken (73) in Verbindung steht, wobei der Nockenfolger (74) ein Nockenende und ein dem Nockenende entgegengesetztes, distales Ende aufweist, eine Feder (75), die an dem entgegengesetzten Ende des Nockenfolgers (74) angeordnet ist, wobei die Feder (75) ein Nockenfolgerende und ein Wellenende aufweist, und eine Welle, die an dem Wellenende angeordnet ist.

5. Tibiaresektionsführung (40) nach Anspruch 4, wobei die Schnittstelle eine Schlüsselschnittstelle ist.

6. Tibiaresektionsführung (40) nach einem der vorhergehenden Ansprüche, wobei der Verriegelungsmechanismus (48) ferner einen visuellen Indikator umfasst, um eine Position des Verriegelungsmechanismus (48) anzuzeigen.

7. Tibiaresektionsführung (40) nach einem der vorhergehenden Ansprüche, wobei die mehreren Stiftlöcher (33) ferner +2mm-Stiftlöcher (33) und Standard-Stiftlöcher (33) umfassen.

8. Tibiaresektionsführung (40) nach einem der Ansprüche 1 bis 7, wobei die mehreren Stiftlöcher (33) ferner ein Aufnahmeloch für einen divergierenden Fixierungsstift (35) umfassen.

## Revendications

1. Guide de résection tibiale (40) comprenant :
un corps (40a) définissant un évidement de pivotement (78) s'étendant latéralement dans le corps (40a), et une fente de résection généralement linéaire (46) disposée au-dessus de l'évidement de pivotement (78) ;
de multiples trous de broche (33) s'étendant antéro-postérieurement à travers le corps (40a) ; et
un ensemble de pivotement (55) disposé à l'intérieur de l'évidement de pivotement (78), l'ensemble de pivotement (55) comprenant un ou plusieurs organes d'engagement tibial complémentaires (68, 68a, 68b),
dans lequel l'ensemble de pivotement (55) est dimensionné de manière ajustée pour tourner axialement à l'intérieur de l'évidement de pivotement (78) par rapport au corps (40a),
**caractérisé en ce que** l'ensemble de pivotement (55) est disposé au-dessus d'un évidement de mécanisme de verrouillage (79) du corps (40a) qui est dimensionné pour enfermer étroitement un mécanisme de verrouillage (48) compris dans le guide tibial (40),
les organes d'engagement tibial complémentaires (68, 68a, 68b) comprennent
(i) une fente (68b) dimensionnée pour recevoir une languette de liaison (64) d'une plaque à pointes (67) fixée sur une partie antérieure d'un tibia,
(ii) et/ou des trous d'engagement tibial (68a) dimensionnés pour recevoir des broches de liaison tibiales (27) fixées sur une partie antérieure du tibia, et
le mécanisme de verrouillage (48) peut être verrouillé pour empêcher l'ensemble de pivotement (55) de tourner axialement.

2. Guide de résection tibiale (40) selon la revendication 1, dans lequel la rotation axiale à l'intérieur de l'évidement de pivotement (78) permet au corps (40a) d'être tourné par rapport à l'ensemble de pivotement (55) pour définir l'angle de pente postérieure souhaité (θ) dans une plage de moins trois degrés à plus 10 degrés par rapport aux broches de liaison tibiales (27).

3. Guide de résection tibiale (40) selon l'une quelconque des revendications précédentes, dans lequel le mécanisme de verrouillage (48) présente une configuration verrouillée et une configuration déverrouillée, et dans lequel le mécanisme de verrouillage (48) est configuré pour verrouiller le guide de résection tibiale (40) à un angle de pente postérieure souhaité (θ) dans la configuration verrouillée.

4. Guide de résection tibiale (40) selon la revendication 3, dans lequel le mécanisme de verrouillage (48) comprend : une came (73), une interface, l'interface communiquant en rotation avec la came (73), un suiveur de came (74) communiquant linéairement avec la came (73), le suiveur de came (74) ayant une extrémité de came et une extrémité opposée distale par rapport à l'extrémité de came, un ressort (75) disposé sur l'extrémité opposée du suiveur de came (74), le ressort (75) ayant une extrémité de suiveur de came et une extrémité d'arbre, et un arbre disposé au niveau de l'extrémité d'arbre.

5. Guide de résection tibiale (40) selon la revendication 4, dans lequel l'interface est une interface à clé.

6. Guide de résection tibiale (40) selon l'une quelconque des revendications précédentes, dans lequel le mécanisme de verrouillage (48) comprend en outre un indicateur visuel pour indiquer une position du mécanisme de verrouillage (48).

7. Guide de résection tibiale (40) selon l'une quelconque des revendications précédentes, dans lequel les multiples trous de broche (33) comprennent en outre des trous de broche +2mm (33) et des trous de broche standard (33).

8. Guide de résection tibiale (40) selon l'une quelconque des revendications 1 à 7, dans lequel les multiples trous de broche (33) comprennent en outre un trou de réception de broche de fixation divergente (35).
